# EUROPEAN PATENT APPLICATION

(11) **EP 4 198 044 A1**
(43) Date of publication of application: **21.06.2023**
(21) Application number: 21216118.6
(22) Date of filing: 20.12.2021
(51) Int. Cl.: C07K 14/315, A61K 35/66, A61K 35/744, C12N 1/20

(54) **AN ISOLATED S. SALIVARIUS STRAIN AND ITS USE AS AN ANTIMICROBIAL PRODUCING PROBIOTIC**

(71) Applicant: Munster Technological University, Bishopstown T12 P928 Cork (IE); TEAGASC, The Agriculture and Food Development Authority, Carlow R93 XE12 (IE)
(72) Inventor: Guinane, Caitriona, Co. Cork (IE); Cotter, Paul, Co. Cork (IE); Begley, Maire, Co. Limerick (IE); Lawrence, Garreth, Co. Tipperary (IE)
(74) Representative: Purdylucey Intellectual Property

(57) **Abstract**

A *Streptococcus salivarius* DPC6487 strain as deposited with the National Collection of Industrial Food and Marine Bacteria under the Accession No. NCIMB 43881.

## Description

### Field of the invention

The invention relates to bacteriocin-producing bacteria as biotherapeutics. More specifically, the invention relates to an isolated *S. salivarius* strain and its antimicrobial peptide Nisin G. The invention also relates to the isolated strain and/or its peptide for use to treat or prevent *Fusobacterium* infections.

### Background of the invention

The human gut microbiome comprises trillions of microbes that coexist and indeed, compete for essential resources that determine their survival. Co-evolution and microbial competition have resulted in the development of several mechanisms that aid in their survival, including the secretion of antimicrobial peptides The gut microbiome is regarded as a reservoir of antimicrobial peptides, such as bacteriocins that may hold potential therapeutic application. Bacteriocins are ribosomally-synthesized antimicrobial peptides, some of which display a broad-spectrum of activity and others a narrow-spectrum of activity. As antibiotic resistant pathogens continue to emerge, bacteriocins represent potential antimicrobial alternatives. Furthermore, bacteriocin-producing probiotic bacteria are of particular interest as they may be utilized to target disease associated taxa *(*Lawrence et al., (2020) International journal of molecular sciences, 21(3), 924.).

The genus *Streptococcus* is well-known for its bacteriocin-producing potential, with lantibiotics being most prevalent. Perhaps, one of the most well-known antimicrobial peptides produced by streptococci is nisin. First discovered in *Lactococcus lactis* in 1928, variants (distinct amino acid substitutions compared to nisin A) of the broad spectrum lantibiotic nisin A have since been reported to be produced by *Streptococcus* species, i.e., *Streptococcus uberis* produces nisin variants nisin U and U2, *Streptococcus hyointestinalis* produces nisin H (O'Connor *et al.,* 2015), while *Streptococcus agalactiae* produces nisin P (Garcia-Gutierrez et al., (2020) P. Scientific Reports, 10, 3738,*)*. Nisin exerts its antimicrobial activity through pore formation and inhibition of cell wall biosynthesis. Nisin A was approved as a food preservative in 1953 and in 1988 the World Health Organisation (WHO) granted nisin generally regarded as safe (GRAS) status.

Several strains of *S.* salivarius produce the lantibiotic salivaricin A, with five variants having been identified to date (Wescombe et al., (2006) Applied and environmental microbiology, 72(2), 1459-1466.). Indeed, *S. salivarius* DPC6993 is a salivaricin B and salivaricin A5 co-producer. Notably, *S. salivarius* strain K12, a salivaricin B and salivaricin A2 co-producer with antagonistic activity against the pathogen *Streptococcus pyogenes,* has been developed as a commercial probiotic and has passed rigorous safety assessments for human use (Burton et al., (2006) Applied and environmental microbiology, 72(4), 3050-3053.; Wescombe et al., (2012) Future microbiology, 7(12), 1355-1371.). *S. salivarius* has been generally regarded as a safe species and strains of *S. salivarius* have been shown to benefit human health in numerous clinical trials.

*Fusobacterium nucleatum* is an important human pathogen with a number of associated pathologies in the gastrointestinal (GI) tract, including colorectal cancer (CRC), inflammatory bowel disease (IBD) and appendicitis. It is a gram-negative and strict anaerobe. There have been clear links made between this pathogen and CRC tumour growth and progression and an increased abundance of Fusobacterium species has been identified by profiling in colon cancer relative to healthy tissues Furthermore, increased abundances of *F. nucleatum* correlates with poor patient prognosis. This organism has a number of virulence factors, including FadA, and there is little doubt that reduced numbers in the GI tract are desirable.

Broad spectrum antibiotics, such as metronidazole, can be used to treat *Fusobacterium* infections. However, this broad-spectrum antibiotic can inhibit the growth of other and beneficial members of the resident gut microbiota. New strategies to reduce the growth of pathogenic species, or eliminate them from the gut microbiota, without harming beneficial bacteria in the process are needed. Probiotic bacteria have potential as an alternative therapy to control these undesirable microbes.

Lawrence et al., (Access Microbiology, vol. 2, issue 1, "Development of a microbially derived therapy against Fusobacterium nucleatum) discloses an abstract of a study in which over 16,000 colonies of gastrointestinal origin were screened for activity against *Fusobacterium nucleatum.* This study led to the identification of a faecal isolate with probiotic potential displaying antagonistic activity against *Fusobacterium nucleatum* in cell culture media. This inhibition was subsequently confirmed in a simulated intestinal model.

O'Shea et al., (Characterisation of enterocin and salivaricin producing lactic acid bacteria from the mammalian gastrointestinal tract, FEMS Microbiol Lett 291(2009)) discloses a study that investigated lactic acid bacteria (LAB) isolated form a variety of mammalian intestinal sources with a view to identifying strains with potential for probiotic and other applications in medicine.

It is an objection of the current invention to overcome at least one of the problems associated with the prior art. The invention provides an *S. salivarius* strain and an antimicrobial peptide with a relatively narrow spectrum of activity against pathogenic bacteria, and of note with anti-*Fusobacterium* activity.

### Summary of the invention

The current inventors have surprisingly found a novel intestinal isolate of *S. salivarius,* namely DPC6487, which was isolated from a neonatal faecal sample, and which produces a novel antimicrobial peptide (i.e., a bacteriocin), which is a nisin variant, with anti*-Fusobacterium* activity.

This peptide is named nisin G and it has the following amino acid sequence: ITSYSLCTPGCKTGVLMACHLKTATCNCSIIVSK (SEQUENCE ID NO.1).

The nucleotide sequence enclosing the peptide is as follows:

ATGAGTACAAATGACTTCAACTTGGATCTTGTATCTGTTTCAAAATCAAATGCAGGTGCT
TCAACTCGTATTACAAGTTATTCGCTATGTACACCGGGATGTAAAACTGGTGTATTAATG
GCGTGTCATCTTAAAACTGCTACATGTAACTGTAGTATCATTGTTTCAAAATAA

(SEQUENCE ID NO. 2).

Indeed, nisin G shows a relatively narrow spectrum of activity, inhibiting the *Fusobacterium* spp. and other streptococci species. The original nisin A peptide also has *anti-Fusobacterium* activity but has a wider spectrum of activity inhibiting many gram-positive genera. Therefore, this novel antimicrobial can target the undesirable pathogens without disrupting the gut microbial balance. This is the first nisin variant reported produced by *S. salivarius.*

This strain and its bacteriocin have a potential use as a biotherapeutic in numerous diseases, including the targeting of bacterial pathogens associated with cancer. As the bacteriocin is produced by a gut isolate it is therefore active in the gastrointestinal (GI) tract, in particular the large intestine (colon).

The added inhibition of *Streptococcus* species and lack of activity against *Lactobacillus* spp., provides a role for controlling vaginal pathogens without disrupting the microbial balance.

The current inventors have found that *S. salivarius* DPC6487 has stronger anti-Fusobacterium inhibitor activity in an *in vitro* plate assay compared to other bacteriocin-producing *S. salivarius* strains tested.

Furthermore, a safety assessment of the *S. salivarius* DPC6487 strain of the invention revealed, by *in silico* analysis on a draft genome, that it demonstrates minimal risk to contribute to transferable antibiotic resistance which is a desirable characteristic of biotherapeutic bacteria. As antibiotic resistance genes are widespread in bacteria, including in some known *S. salivarius* strains, the absence of apparent transferable antibiotic resistant genes in the draft genome of the strain of the invention contributes to its potential human use. The current inventors have demonstrated that the antimicrobial-producing *S. salivarius* strain DPC6487 demonstrated inhibitory activity against *F. nucleatum* DSM15643 and *F. peridonticum* DSM19545. A crude preparation of the Nisin G peptide also demonstrated antimicrobial activity against *F. nucleatum* DSM15643. The *S. salivarius* strain DPC6487 also showed inhibitor activity against *S. agalactiace, S. thermophilus* and *S. uberis.* In this study, *S. salivarius* DPC6487 demonstrated increased potency compared to the salivaricin-producing *S. salivarius* DPC6993 against *L. bulgaricus.*

Interestingly, the *S. salivarius* strain DPC6487 demonstrated no inhibitory activity against the species *S. mutans, S. simulans,* or *Clostridioides difficile* and genera *Lactobacillus* (except *Lactobacillus bulgaricus), Listeria* or *Staphylococcus.* Distinct antimicrobial activity is observed with *L. lactis,* a nisin A producer, when targeting these genera. Furthermore, no activity was observed against Gram negative *E. coli* strains, as previously reported for nisin variants.

An aspect of the invention provides a *Streptococcus salivarius* strain characterised in that the strain is isolated from a human, preferably gastrointestinal (GI) tract, and is capable of inhibiting growth of *Fusobacterium spp.,* preferably in the distal colon, and which produces an antimicrobial peptide (herein referred to as "the strain of the invention").

Preferably, the strain is characterised by isolation from a human neonatal gastrointestinal tract.

The antimicrobial peptide is nisin G and it has an amino acid sequence of SEQUENCE ID NO. 1. In an embodiment, the antimicrobial peptide is a peptide variant of SEQUENCE ID NO. 1, having 1 to 3 amino acid changes compared with SEQUENCE ID NO. 1.

In one embodiment, the strain of the invention is *Streptococcus salivarius* DPC6487 strain and variants thereof. The "variant" thereof is one that retains the phenotypical characteristics of bacterium as described herein.

Preferably, the *Fusobacterium spp.* is selected from *Fusobacterium nucleatum and Fusobacterium peridontium.*

Typically, the strain has inhibitory activity against Streptococcus strains, *S. agalactiae, S. thermophilus* and *S. uberis.*

In an embodiment, the strain has no activity against *S. mutans, S. simulans, Clostridioides difficile,* and strains from the genera *Lactobacillus* (except for the sensitive indicator *Lactobacillus delbrueckii* subsp. *bulgaricus*)*, Listeria* and *Staphylococcus.*

A further aspect of the invention provides a bacteriocin, or antimicrobial peptide, expressed by the strain of the invention.

An aspect of the invention provides an antimicrobial peptide having a sequence of SEQUENCE ID NO. 1 (nisin G), or a variant thereof having 1 to 3 amino acid changes compared with SEQUENCE ID NO. 1 (Nisin G and variants thereof are herein referred to as the "antimicrobial peptide of the invention"). The antimicrobial peptide may be an isolated peptide.

The invention also relates to a nucleic acid encoding the antimicrobial peptide of the invention. Typically, this nucleic acid comprises (or consists) of SEQUENCE ID NO. 2.

Ideally, the antimicrobial peptide of the invention has antibacterial activity against *Fusobacterium spp.,* and typically, *Fusobacterium nucleatum.*

The invention also provides a composition comprising the antimicrobial peptide of the invention or the strain of the invention. The composition may be a pharmaceutical composition and may optionally comprise one or more suitable pharmaceutical excipients (herein referred to as "the composition of the invention").

The composition of the invention may be formulated as an antibacterial or antibiotic formulation. In this regard an antibacterial or antibiotic formulation comprising the antimicrobial peptide of the invention or strain of the invention is provided.

The composition of the invention may be formulated for topical administration.

Also provided by the invention is a recombinant vector comprising one of more of the nucleic acid of the invention or a nucleic acid encoding the peptide of the invention. The invention also relates to a host cell transformed by a recombinant vector of the invention.

Also provided by the invention is a plasmid comprising a nucleic acid encoding the peptide of the invention.

The invention provides a probiotic comprising the antimicrobial peptide of the invention or the strain of the invention.

The invention provides a food or beverage product or comestible product comprising the antimicrobial peptide of the invention or the strain of the invention.

An aspect of the invention provides the antimicrobial peptide of the invention, the strain of the invention or the composition of the invention, for use as a medicament.

An aspect of the invention provides the antimicrobial peptide of the invention, the strain of the invention or the composition of the invention, for use as an antibacterial agent or an antibiotic.

An aspect of the invention provides the antimicrobial peptide of the invention, the strain of the invention or the composition of the invention for use in treating or preventing a disease or condition characterised by growth of *Fusobacterium* spp.

Typically, the disease or condition is one associated with the gastrointestinal (GI) tract, preferably the distal colon

In an embodiment, the disease or condition is one or more selected from the group comprising colorectal cancer (CRC), inflammatory bowel disease (IBD) and appendicitis.

In an embodiment, the disease or condition is a periodontal disease. The periodontal disease may be selected from gingivitis and periodontitis.

The invention also relates to the strain of the invention, the antimicrobial peptide of the invention, or a transformed host of the invention, for use as a probiotic culture.

An aspect of the invention provides the antimicrobial peptide of the invention, the strain of the invention or the composition of the invention for use in treating or preventing a disease or condition characterised by growth of *S. agalactiae* in a subject. Typically, the subject is a pregnant human and/or an immunocompromised subject.

The disease or condition to be treated or prevented may be a vaginal infection. The disease or condition to be treated or prevented may bacterial vaginosis.

### Definitions and General Preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

Preferably, in this context the "disease" to be treated or prevented is a disease or condition characterised by growth of Fusobacterium.

As used herein, the term "treatment" or "treating" refer to an intervention (e.g. the administration of an agent to a subject) which cures, ameliorates or lessens the symptoms of a disease or removes (or lessens the impact of) its cause(s). In this case, the term is used synonymously with the term "therapy". It can be manifested by a permanent or temporary improvement in the subject's condition. In this context it includes limiting and/or reversing disease progression.

As used herein the terms "prevention" or "preventing" refer to an intervention (e.g. the administration of an agent to a subject), which prevents or delays the onset or progression of a disease, or the severity of a disease, in a subject, or reduces (or eradicates) its incidence within a treated population.

When used herein, the term "composition" should be understood to mean something made by the hand of man, and not including naturally occurring compositions. Compositions may be formulated in unit dosage form, i.e., in the form of discrete portions containing a unit dose, or a multiple or sub-unit of a unit dose.

When used herein, the term "pharmaceutical composition" may comprise one or more pharmaceutically acceptable diluents, excipients or carriers. Even though the peptides and compositions of the present invention can be administered alone, they will generally be administered in admixture with a pharmaceutical carrier, excipient or diluent, particularly for human therapy. The pharmaceutical compositions may be for human or animal usage in human and veterinary medicine. Examples of such suitable excipients for the various different forms of pharmaceutical compositions described herein may be found in the "Handbook of Pharmaceutical Excipients", 2nd Edition, (1994), edited by A Wade and PJ Weller. In particular, formulations for topical delivery are described in "Topical Drug Delivery Formulations" edited by David Osborne and Antonio Aman, Taylor & Francis, the complete contents of which are incorporated herein by reference. Acceptable carriers or diluents for therapeutic use are well known in the pharmaceutical art, and are described, for example, in Remington's Pharmaceutical Sciences, Mack Publishing Co. (A. R. Gennaro edit. 1985).

In the specification, "pharmaceutically acceptable" means approved by a regulatory agency of the Federal or a state government or listed in the U.S. Pharmacopeia or other generally recognized pharmacopeia for use in animals, and more particularly in humans.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Preferably, the excipient is one or more non-natural excipients.

The term "carrier" refers to a diluent, adjuvant, excipient, or vehicle with which the composition is administered. Examples of suitable carriers include lactose, starch, glucose, methyl cellulose, magnesium stearate, mannitol, sorbitol and the like. Examples of suitable diluents include ethanol, glycerol and water. Pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

Saline solutions and aqueous dextrose and glycerol solutions can also be employed as liquid carriers. Suitable excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The term "peptide" used herein refers to a polymer, generally composed of up to 50 amino acids, for example 5 to 50 amino acid monomers typically linked via peptide bond linkage. Peptides (including fragments and variants thereof) of and for use in the invention may be generated wholly or partly by chemical synthesis or by expression from nucleic acid. For example, the peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984); and M. Bodanzsky and A. Bodanzsky, The Practice of Peptide Synthesis, Springer Verlag, New York (1984)). When necessary, any of the peptides employed in the invention can be chemically modified to increase their stability. A chemically modified peptide or a peptide analog includes any functional chemical equivalent of the peptide characterized by its increased stability and/or efficacy *in vivo* or *in vitro* in respect of the practice of the invention.

As used herein, the term "effective amount or a therapeutically effective amount" as applied to the invention defines an amount that can be administered to a subject without excessive toxicity, irritation, allergic response, or other problem or complication, commensurate with a reasonable benefit/risk ratio, but one that is sufficient to provide the desired effect, e.g., inhibition of Fusobacterium. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result need not be a complete cure. A therapeutic result may be a permanent or temporary improvement in the subject's condition.

The term "subject" means a human or animal, more typically a mammal. In one aspect, the subject is a human.

The term "symptom" is defined as an indication of disease, illness, injury, or that something is not right in the body.

In the specification, the term "isolated" should be considered to mean material removed from its original environment in which it naturally occurs, for example, in this instance a bacterial strain of the mammalian gut and/or an antimicrobial peptide. The removed material is typically cultivated, purified and cultured separately from the environment in which it was located. Thus, the purified isolated bacterial strain in this instance ideally does not contain any significant amounts of other bacterial strains. The isolated strain or variant of the invention may be provided in a viable or non-viable form, and in a culturable or non-culturable form. The invention also relates to an isolated strain of the invention, or variant thereof, of an antimicrobial peptide, in any format, for example a freeze-dried form, a suspension, a powder, or a broth, for example a fermentation broth or an extract from a fermentation broth that is enriched in the antimicrobial peptide of the invention.

The term "freeze-dried form" should be understood to mean that the strain, the antimicrobial peptide of the invention, optionally together with other ingredients including, for example, preservatives, is frozen and then the ice crystals in the frozen strain are sublimated under vacuum.

In the specification, the term "mammal" or "individual" as employed herein should be taken to mean a human.

The term "comestible product" should be understood to include products that are intended to be consumed by ingestion by humans or animals, such as foods and drinks. In particular, the comestible product is a food or drink product intended for consumption by humans, for example a fermented product or a diary product, especially a fermented dairy product such as a yoghurt.

The term "variant" as applied to *Steptococcus salivarius* DPC6487 is one that retains the phenotypical characteristics of the bacterium as described herein and that expresses an antimicrobial peptide of SEQUENCE ID NO. 1, or a variant thereof, having antibacterial activity against *Fusobacterium spp* and preferably *F. nucleatum.* Preferably, the term variant should be understood to mean progeny (unmodified descendants), modified descendants, or derivatives of *Steptococcus salivarius* DPC6487, for example strains which are genetically modified to alter the genotype of the bacteria, or strains which are altered by natural processes such as selection or serial passage. Typically, the variant of the strain is one isolated from the neonatal GI tract, and preferably neonatal faeces. However, it will be appreciated that the strain and nisin it produces may be found in other environments or areas of the body. The variant generally has a 16S rRNA fragment gene sequence (i.e. a partial 16S rRNA gene sequence) that is identical or substantially identical with the strain of the invention, such as the deposited strain *Steptococcus salivarius* DPC6487, for example at least 98%, 98.5%, 99%, 99.1%, at least 99.1%, 99.2%, 99.3%, 99.4%, 99.5%, 99.6%, 99.7%, 99.8% or 99.9% identical. Sequence identity can be determined using an online algorithm "BLAST", publicly available at http://www.ncbi.nlm.nih.gov/BLAST/, or EMBOSS Needle (http://www.ebi.ac.uk/tools/psa/emboss_needle/).

In this specification, the term "sequence identity" should be understood mean the amount of nucleotides which match between different sequences. For example, a 16S rRNA gene sequence that shares at least 98% sequence identity with a reference sequence is one in which any 98% of aligned nucleotides of the variant are identical to the corresponding nucleotides in the reference sequence across the entire length of the sequence. Sequence identity is the amount of characters which match exactly between two different sequences. Hereby, gaps are not counted, and the measurement is relational to the shorter of the two sequences

The term "variant" as applied to the Nisin G peptide having the amino acid sequence of SEQUENCE ID NO. 1 should be understood to mean a peptide comprising or consisting of a sequence having 1 to 4, preferably 1 to 3, amino acid changes or alterations compared with SEQUENCE ID NO. 1. The alterations involve insertion, addition, deletion and/or substitution of 3 or fewer amino acids, more preferably of 2 or fewer amino acids, most preferably 1 amino acid only. The variant may have conservative amino acid changes, wherein the amino acid being introduced is similar structurally, chemically or functionally to that being substituted. The term "variant" is also intended to include chemical derivatives of the protein, i.e., where one or more residues is chemically derivatized by reaction of a functional side group. Also included are variants in which naturally occurring amino acid residues are replaced with amino acid analogues. Details of amino acid analogues are well known to those skilled in the art. The peptide variant will generally have less than 50 residues, 40 residues or 35 residues. The variant may have 34 residues. The variant is one which maintains the antimicrobial activity of nisin G. In an embodiment, the variant is one that comprises one or more of Gly18Ala, Asp20His and His31Ile compared with the peptide sequence of Nisin A. In other words, in an embodiment the variant does not have an amino acid change at position 18, position 20 and/or position 31. This variant would comprise at least alanine (Ala) at position 18 of SEQUENCE ID NO.1, histidine (His) at position 20 of SEQUENCE ID NO. 1 and/or isoleucine (Ile) at position 31 of SEQUENCE ID NO. 1.

When used herein the term "colony forming unit or CFU" refers to a unit used to estimate the number of bacterial cells in a sample which are viable.

### Brief Disclosure of the Figures

The invention will be more clearly understood from the following description of an embodiment thereof, given by way of example only, with reference to the accompanying drawings, in which:

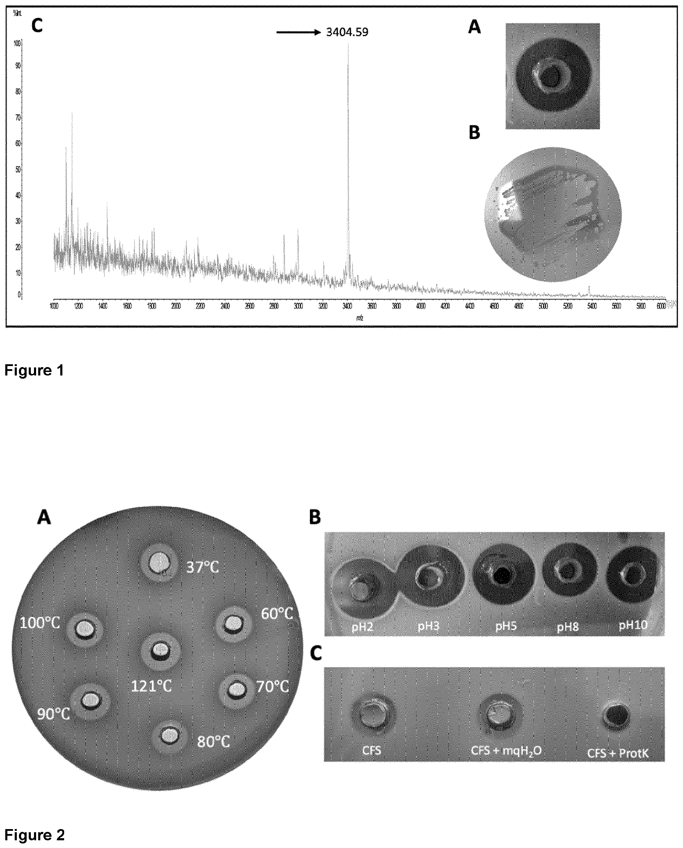

**Figure 1****:** A antimicrobial is produced by *S. salivarius* DPC6487. CFS of *S. salivarius* DPC6487 demonstrated antimicrobial activity against *L. delbrueckii ssp. bulgaricus* DPC5383 in a WDA **(A)**. Deferred antagonism assay whereby *S.* salivarius DPC6487 demonstrated antimicrobial activity against *F. nucleatum* DSM15643 **(B).** The presence of a 3404.59 Da mass was revealed by MALDI-TOF MS **(C).**
**Figure 2****:** Effect of heat **(A)**, pH **(B)** and proteinase K **(C)** on the CFS of *S. salivarius* DPC6487. After subjection to heat, pH and proteinase K the antimicrobial activity of *S. salivarius* DPC6487 CFS was assessed against *L. delbrueckii ssp. bulgaricus* DPC5383.

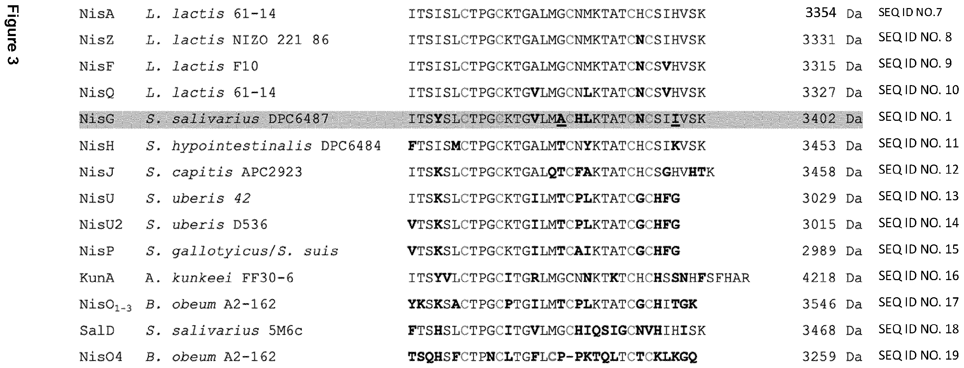

**Figure 3****:** Sequence alignment of natural nisin (Nis) variants. Amino acid substitutions are highlighted in bold; cysteines are highlighted in green and nisin G (NisG) is shaded. Amino acid substitutions unique to NisG are underlined. Salivaricin D (SalD) produced by *S. salivarius* 5M6c and Kunkecin A (KunA) produced by *Apilactobacillus kunkeei* FF30-6 were included as they are considered 'nisin-like'. Nisin accession (reference is provided where accession is not linked to primary source): Nisin A, ABN45880; Nisin Z, ABV64387; Nisin F, ABU45463; Nisin Q, ADB43136; Nisin H, AKB95119; Nisin J, (O'Sullivan et al., 2020); Nisin U, Q2QBT0; Nisin U2, ABO32538; Nisin P, (Zhang et al., 2012); Nisin O, (Hatziioanou et al., 2017); Kunkecin A (Zendo et al., 2020), Salivaricin D, AEX55166.

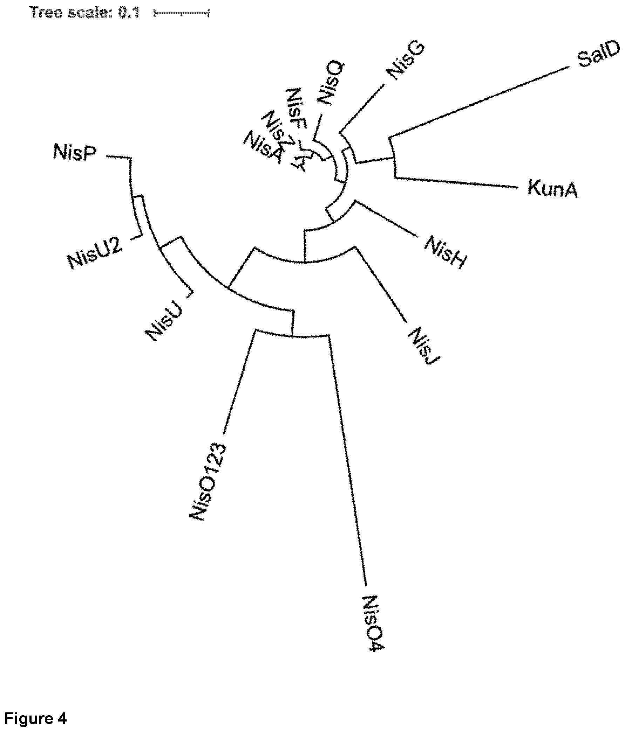

**Figure 4****:** Phylogenetic relationship of nisin G to other natural nisin variants. The phylogenetic relationship was inferred using the neighbourhood-joining method (Saitou & Nei, 1987); evolutionary distances were computed using the Poisson correction method. All steps involved in the construction of the tree were performed in MEGAX (Kumar et al., 2018) and visualised using ITOL (Letunic & Bork, 2019). Branch length (Scale = 0.1) indicates the number of amino acid substitutions per site. Nis, Nisin; Sal, salivaricin; Kun, Kunkecin.

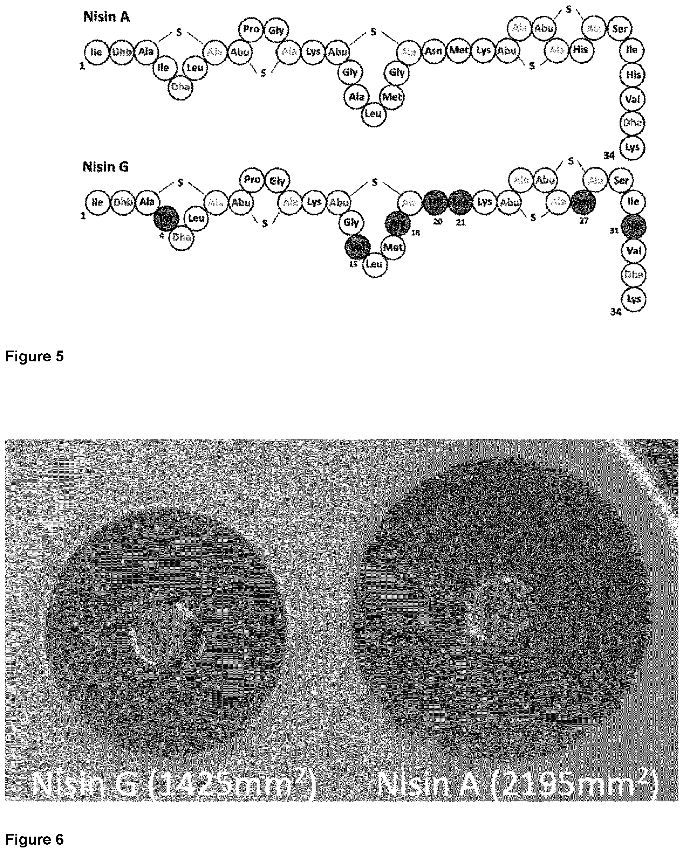

**Figure 5****:** Nisin G gene cluster found in the genome of *S. salivarius* DPC6487 Amino acid changes in nisin G are indicated in red and substituted positions compared to nisin A are numbered. Posttranslational modifications are represented by Dha (dehydroalanine), Dhb (dehydrobutyrine), and Abu-S-Ala (3-methyllanthionine).
**Figure 6****:** Antimicrobial activity of nisin G purified from *S. salivarius* DPC6487 and nisin A purified from *L. lactis* NZ9700 against *L. bulgaricus* DPC5383. Both nisin G and nisin A were assayed at a concentration of 261mM by WDA. Zones of inhibition are indicated in brackets and were calculated as the area of zone of inhibition (πr2) - area of well (πr2) in millimeters.

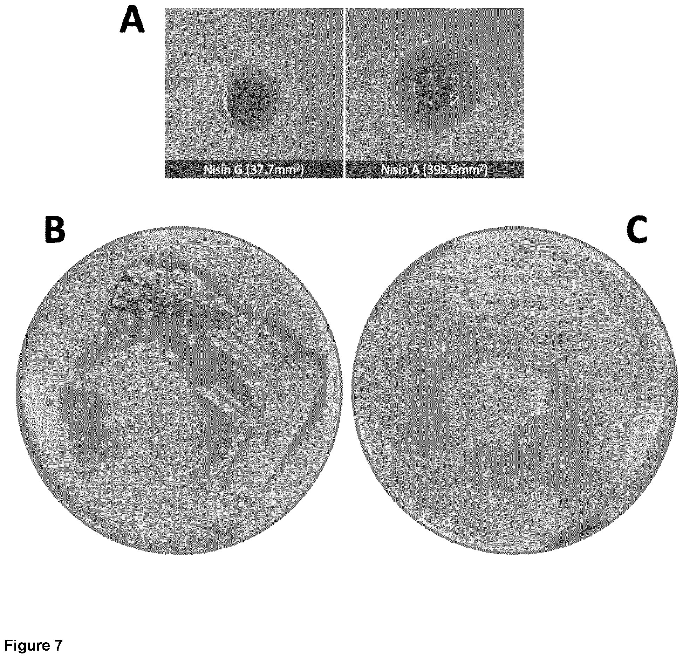

**Figure 7****:** Activity of purified nisin G and nisin A against *F. nucleatum* DSM15643 by WDA **(A)** and the antimicrobial activity of nisin G-producing *S. salivarius* DPC6487 **(B)** and nisin A-producing *L*. *lactis* NZ9700 **(C)** against *F. nucleatum* DSM15643 by deferred antagonism assay. Zones of inhibition for purified nisin peptides are indicated in brackets and were calculated as the area of zone of inhibition (πr2) - area of well (πr2) in millimeters.

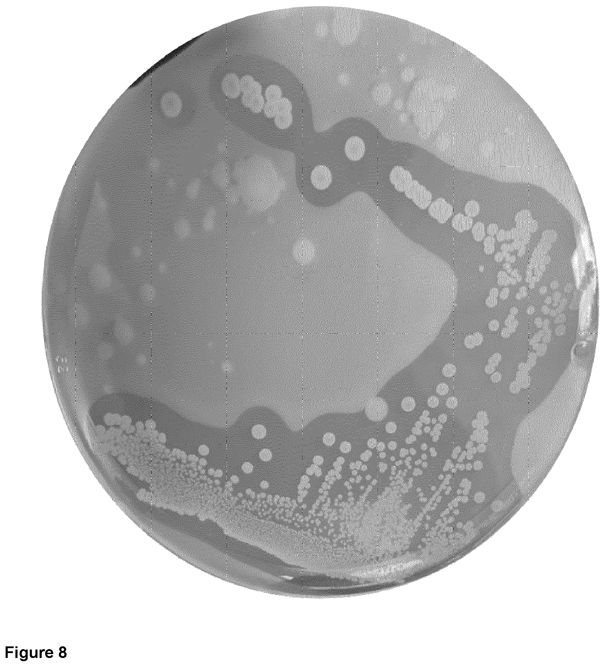

**Figure 8****:** *S*. *salivarius* DPC6487 activity against *S. agalactiae* ATCC13813.

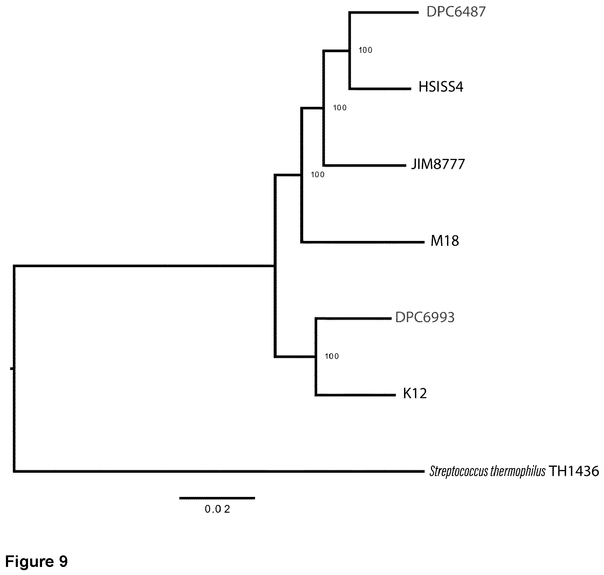

**Figure 9****:** Phylogenetic tree based on whole genome comparisons of *S. salivarius* DPC6487 (blue) and reference genomes of *S. salivarius* using the RAxML method within the codon tree pipeline at PATRIC. Numbers at nodes indicate confidence values (%) of 100 rounds of bootstrapping. Bar, number of substitutions per site. *Streptococcus thermophilus* TH1436 (Accession no. AYTT00000000) was used as the outgroup taxa.

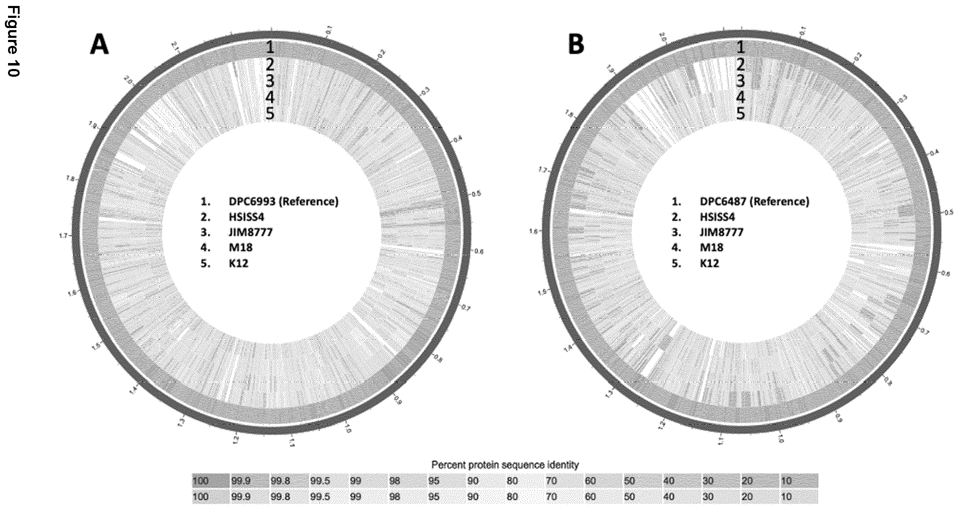

**Figure 10****:** Proteome comparison of *S. salivarius* DPC6487 **(B)** with *S. salivarius* genomes HSISS4, JIM8777, M18 and K12. Each proteome comparison is presented as a closed circle. Changes in conservation of each comparative strain relative to each reference strain are indicated where blue representing the highest protein similarity and red representing lowest protein similarity. Gaps may indicate deletions.

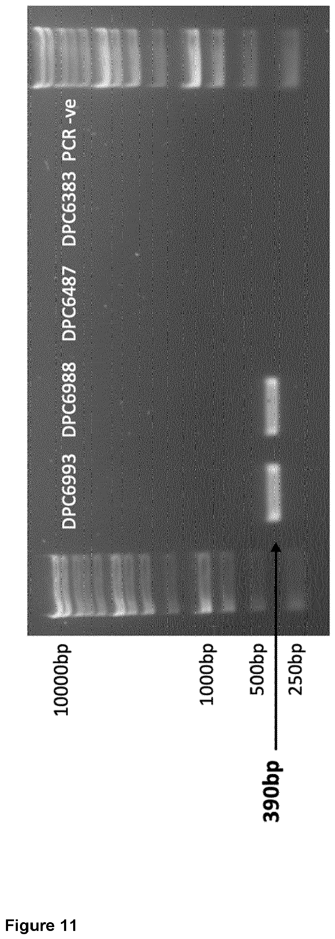

**Figure 11****:** Absence of gene *mef*E in erythromycin sensitive strains *S. salivarius* DPC6487 and DPC6383.

### Detailed Disclosure of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

The invention provides a *Streptococcus salivarius* strain characterised in that the strain was isolated from the human, preferably neonatal gastrointestinal tract, produces an antimicrobial peptide Nisin G having the amino acid sequence of SEQUENCE ID NO. 1 and which is capable of inhibiting growth of *Fusobacterium spp.*

Notably, the strain of the invention is *S. salivarius* DPC6487 as deposited with the National Collection of Industrial Food and Marine Bacteria (NCIMB at Ferguson Building, Craibstone Estate, Bucksburn, Aberdeen, AB21 9YA Scotland) under the Accession No. NCIMB 43881 on 3 November 2021 (deposited in the name of Teagasc Food Research Centre, Moorepark, Fermoy, Co. Cork, Ireland). The strain was deposited under the Budapest Treaty on the International Recognition of the Deposit of Microorganisms for the purposes of patent procedure.

The strain of the invention is a gram positive, facultative anaerobe.

The strain of the invention has a partial 16S rRNA sequence defined by SEQUENCE ID NO. 4. This is a 16S rRNA fragment. Variants of the strain of the invention may comprise a partial 16S rRNA sequence that is identical or substantially identical with the strain of the invention for example at least 98%, 98.5%, 99%, 99.1%, at least 99.1%, 99.2%, 99.3%, 99.4%, 99.5%, 99.6%, 99.7%, 99.8% or 99.9% identical to SEQUENCE ID NO. 4.

SEQUENCE ID NO 4 has the following sequence:

tttatttgaaaggggcaattgctccactacaagatggacctgcgttgtattagctagtaggtgaggtaacggctcacctaggcgac
gatacatagccgacctgagagggtgatcggccacactgggactgagacacggcccagactcctacgggaggcagcagtag
ggaatcttcggcaatgggggcaaccctgaccgagcaacgccgcgtgagtgaagaaggttttcggatcgtaaagctctgttgtaa
gtcaagaacgagtgtgagagtggaaagttcacactgtgacggtagcttaccagaaagggacggctaactacgtgccagcagc
cgcggtaatacgtaggtcccgagcgttgtccggatttattgggcgtaaagcgagcgcaggcggtttgataagtctgaagttaaag
gctgtggctcaaccatagttcgctttggaaactgtcaaacttgagtgcagaaggggagagtggaattccatgtgtagcggtgaaat
gcgtagatatatggaggaacaccggtggcgaaagcggctctctggtctgtaactgacgctgaggctcgaaagcgtggggagc
gaacaggattagataccctggtagtccacgccgtaaacgatgagtgctaggtgttggatcctttccgggattcagtgccgcagcta
acgcattaagcactccgcctggggagtacgaccgcaaggttgaaactcaaaggaattgacgggggcccgcacaagcggtgg
agcatgtggtttaattcgaagcaacgcgaagaaccttaccaggtcttgacatcccgatgctatttctagagatagaaagttacttcg
gtacatcggtgacaggtggtgcatggttgtcgtcagctcgtgtcgtgagatgttgggttaagtcccgcaacgagcgcaacccctatt
gttagttgccatcattcagttgggcactctagcgagactgccggtaataaaccggaggaaggtggggatgacgtcaaatcatcat
gccccttatgacctgggctacacacgtgctacaatggttggtacaacgagttgcgagtcggtgacggcaagctaatctcttaaag
ccaatctcagttcggattgtaggctgcaactcgcctacatgaagtcggaatcgctagtaatcgcggatcagcacgccgcggtgaa
tacgttcccgggccttgtacacaccgcccgtcacaccacgagagtttgtaacacccgaagtcggtgaggtaaccttttggagcca
gccgcctaaggtgggatagatgattggggtgaagtcgtaacaaggtagccgtatcggaaggtgcggctggatcacctcctt

*S. salivarius* DPC6487 exhibited a narrower spectrum of activity compared to the nisin A producer *Lactococcus lactis* NZ9700, with activity in an overlay assay against the *Fusobacterium* strains and the other streptococci tested. Such narrow spectrum activity is desirable in the context of not causing collateral damage to gut commensals

Preferably, the *Fusobacterium spp.* inhibited is selected from *Fusobacterium nucleatum and Fusobacterium peridontium.* Typically, the strain or the antimicrobial peptide of the invention also have inhibitory activity against Streptococcus strains, *S. agalactiace, S. thermophilus* and *S. uberis.*

Notably, the strain or the antimicrobial peptide of the invention have no activity against strains tested of *Streptococcus mutans, Streptococcus simulans, Clostridioides difficile,* and strains tested from the genera *Lactobacillus, Listeria* and *Staphylococcus.*

The *Lactobacillus* strains tested included strains of *Lactobacillus fermentum* and *Lactobacillus plantarum.* The *Listeria* strains tested were *Listeria monocytogenes* and *Listeria innocua.* The *Staphylococcus* strain tested was *Staphylococcus aureus* including a methicillin resistant strain.

The strain of the invention and its antimicrobial peptide have a use as a biotherapeutic in numerous diseases.

The disease may be one associate with or caused by *Fuscobacterium* spp. such as *F. nucleatum.* The disease may be one associated with the GI tract and can include but is not limited to colorectal cancer (CRC), inflammatory bowel disease (IBD) and appendicitis.

CRC-associated *F. nucleatum* represents a potential therapeutic target and eradicating or suppressing the growth of this pathogen within the human gut microbiome may ultimately contribute to reducing or removing the overall risk of disease development.

The disease may be a periodontal disease, such as gingivitis or periodontitis. *F. nucleatum* and *F. periodonticum* are also oral pathogens

Interestingly, the strain also has inhibitory activity against *S. agalactiae.* Thus, an aspect of the invention relates to treatment and prevention of a disease or condition characterised by growth of or caused by *S. agalactiae.* As invasive infections caused by *S. agalactiae* have been reported in pregnant women, new-borns and in adults with immunosuppressive diseases such as cancer and HIV, this finding suggests an application for the strain of the invention, particularly *S. salivarius* DPC6487, to reduce the risk of *S. agalactiae* infection.

The disease or condition to be treated or prevented may be a vaginal infection. The disease or condition to be treated or prevented may bacterial vaginosis.

*Lactobacillus* spp are the predominant coloniser of the vaginal tract and plays an essential role in preventing the invasion of pathogenic bacteria. Therefore, as the strain of the current invention or its antimicrobial peptide have no inhibitory activity against *Lactobacillus spp.,* the current invention can be used to target pathogenic bacteria such as *S. agalactiae,* while leaving these beneficial bacteria unharmed.

Bacterial vaginosis is a relevant indication and is characterized by replacement of vaginal *lactobacilli* with predominantly anaerobic microorganisms such as *Gardnerella vaginalis* and *Prevotella, Peptostreptococcus and Bacteroides spp.*

Preferably, an effective amount of the strain, or peptide, or composition is used in the medical use or method of the invention.

The strain of the invention demonstrates minimal risk to contribute to antibiotic resistance, due to the absence of apparent transferable antibiotic resistance genes. Of note, the strain of the invention produces a novel bacteriocin, or antimicrobial peptide, called Nisin G.

The genetic loci of the nisin gene cluster were analysed and the antimicrobial peptide produced was identified and characterised by the current inventors. Whole genome sequencing of *S. salivarius* DPC6487 confirmed the presence of a gene encoding a novel nisin variant designated nisin G. The structural nisin G peptide has 7 amino acid differences relative to prototypical nisin A (Ile4Tyr, Ala15Val, Gly18Ala, Asn20His, Meth21 Leu, His27Asn and His31 Ile). Nisin G demonstrates three unique amino acid variations when compared to all natural nisin variants, specifically Gly18Ala, Asp20His and His31Ile.

The precursor comprising the peptide leader for Nisin G has the following amino acid sequence:
MSTNDFNLDLVSVSKSNAGASTRITSYSLCTPGCKTGVLMACHLKTATCNCSIIVSK (SEQUENCE ID NO.3)

Purified nisin G and nisin A both demonstrated antimicrobial activity against *F. nucleatum* DSM15643. Nisin G is capable of inhibiting the activity of *F. nucleatum* at a concentration of 261mM.

The composition of the invention may be solid or liquid. The composition may comprise a carrier for oral delivery. The carrier may be in the form of tablet, capsule, powder, granules, microparticles or nanoparticles. The carrier may be configured for targeted release in the intestine (*i.e.,* configured for gastric transit and distal colon or ileal release).

The composition of the invention may be dried or lyophilised.

The composition of the invention may be provided in a unit dose form suitable for oral and/or topical administration, *i.e.,* a tablet, a capsule, a pellet, freeze-dried granules or powder, spray-dried granules or powder, nanoparticles, microparticles or in a liquid form.

The composition may be a comestible product. The composition may be a food or beverage product, a food or beverage additive, a nutritional supplement, or an animal feed or drink additive (for example, in animal feed or pet food, or liquid refreshments (water, supplementary milk, and the like)). The products and additives are suitable for human ingestion and tailored to be suitable for non-human mammal and animal ingestion. The beverage in this instance can be drinking water, supplementary milk, fermented milk or other liquid drinks provided to the mammal or animal.

The strain in the composition may be viable or non-viable and may comprise a strain extract (*i.e.,* bacterial cell lysate) or supernatant derived from the strain. The extract or supernatant may be in any physical form, for example liquid or dried. The extract or supernatant may comprise or otherwise express the peptide of the invention.

The invention also provides a method of producing a supernatant from the strain of the invention, preferably, *S*. *salivarius* DPC6487 comprising a step of culturing the isolated strain and separating the supernatant from the strain.

The invention also provides a method of producing an extract from an isolated strain of the invention, preferably *S*. *salivarius* DPC6487 comprising a step of lysing the cell and separating the cell extract from lysed cell material.

The invention also provides a supernatant or bacterial material or extract (for example a cell lysate) formed according to the method of the invention.

The composition, if desired, can also contain minor amounts of wetting or emulsifying agents, or pH buffering agents.

The composition may comprise between 10³ and 10¹² cfu of the strain of the invention per gram of dry weight of the composition. The composition may comprise at least 10⁶ cfu per g of composition. In one aspect, the composition may comprise 1 × 10⁹ spores/ml or cfu/ml. Typically, the composition may comprise between 1 × 10⁶ to 1 × 10¹⁰ spores/ml or cfu/ml. For example, 1 × 10¹⁰ spores/ml or cfu/ml, 1 × 10⁷ spores/ml or cfu/ml, 1 × 10⁸ spores/ml or cfu/ml, 1 × 10⁹ spores/ml or cfu/ml, or 1 × 10¹⁰ spores/ml or cfu/ml.

It is preferable that the strain or composition is administered at least one per week over at treatment period of at least 4 weeks, and preferably at least 5, 6, 7, 8, 9, 10, 11, 12, 14, 16, 18 or 20 week period. Preferably, the strain or composition is administered several times a week, and ideally once a day.

The composition of the invention may comprise one or more dietary fibres and/or a prebiotic.

The formulation may also be a hygiene product, for example an antibacterial formulation, or a fermentation product such as a fermentation broth. For formulations that comprise the antimicrobial peptide of the invention, it will be appreciated that the peptide may be directly added to the formulation, or it may be produced *in-situ* in the formulation by a bacteria, for example the isolated strain of the invention or a variant thereof.

In one aspect, when the composition is suitable for topical administration, the composition comprises a carrier for topical administration selected from the group comprising powder, granules, microparticles, nanoparticles, a cream, a spray, spores, or liquid. The powder, granules, microparticles, nanoparticles and spray, as well as the spores themselves, can all be freeze-dried or spray-dried prior to application. Proteins and polypeptides (including variants and fragments thereof) of and for use in the invention may be generated wholly or partly by chemical synthesis or by expression from nucleic acid. The proteins and peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984)).

The invention also relates to a recombinant vector comprising a nucleic acid encoding an antimicrobial peptide of the invention. The nucleic acids may be cloned as separate entities (i.e. distinct nucleic acid constructs), or in a same construct, under distinct promoter regions or in a single operon. Typically, the nucleic acids are cloned into a recombinant vector (for example a plasmid) which is capable of replicating in the host bacteria. Typical plasmids contain, in addition to the cloned insert, a selection gene (i.e. antibiotic resistance, a dye etc) and an origin of replication effective in the host bacterium. The plasmid may also comprise regulatory sequences, for example promoters, terminators and/or enhancers. Examples of such vectors are pNZ44 (McGrath S, Fitzgerald GF, van Sinderen D (2001) Improvement and optimization of two engineered phage resistance mechanisms in Lactococcus lactis. Appl. Environ. Microbiol. 67 (2): 608-616)) and pCl372 (Hayes F, Daly C, Fitzgerald GF (1990) Identification of the Minimal Replicon of Lactococcus lactis subsp. lactis UC317 Plasmid pCl305. Appl. Environ. Microbiol. 56: 202-209)). However, any recombinant vector suitable for replicating in a host bacteria known to the person skilled in the art may be used.

The nucleic acid may also be cloned into an integrative cassette suitable for integration into the genome of suitable host bacteria. Such an integrative cassette typically comprises a nucleic acid encoding an antimicrobial peptide of the invention or a cognate immunity protein of the invention, or both, linked to (or flanked by) one or several sequences allowing integration, preferably site-specific integration. Such sequences may be for instance nucleic acid sequences homologous to a targeted region of the genome, allowing integration through crossing over. Various techniques can be used to insert a nucleic acid into a host bacteria, for example through natural transformation or electroporation.

The host bacteria suitable for cloning the antimicrobial peptide and/or the cognate immunity protein may be selected from any host bacteria known to a person skilled in the art such as, for example, *Lactococcus, Lactobacillus and Enterococcus.*

A further aspect of the invention a substantially pure culture of the strain of the invention.

A further aspect of the invention provides a method of growing the strain of the invention in a fermentation reactor. The strain grown in the reactor and the isolated strain are also provided.

A fermentation broth comprising the strain and/or the antimicrobial peptide of the invention are also provided.

An aspect of the invention provides a method for treating or preventing a disease or condition characterised by growth of *Fusobacterium* spp, comprising administering the antimicrobial peptide of the invention, the strain of the invention or the composition of the invention to a subject. Typically, the disease or condition is one associated with the gastrointestinal (GI) tract, preferably the distal colon.

An aspect of the invention provides a method for treating or preventing a disease or condition characterised by growth of *S. agalactiae* in a subject, comprising administering the antimicrobial peptide of the invention, the strain of the invention or the composition of the invention to a subject.

The preferred features and embodiments of the medical uses of the invention also apply to the methods of treatment or preventing of the invention.

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXAMPLES

### EXAMPLE 1

### Streptococcus salivarius DPC6487 produces the novel lantibiotic, nisin G, that inhibits Fusobacterium nucleatum.

### Materials and Methods

### Bacterial strains and cultivation media

*S. salivarius* DPC6487 was cultivated under anaerobic conditions at 37°C in Brain Heart Infusion (BHI, Difco Laboratories, Detroit, MI, USA) broth and agar medium containing 1.5% w/v agar. Anaerobic conditions were simulated using anaerobic jars with Anaerocult A gas packs (Merck, Darmstadt, Germany) or a Don Whitley Anaerobic workstation (nitrogen 85%, carbon dioxide 5%, hydrogen 10%). A full list of bacteria and their culture conditions used in this study are presented in **Table 1.**

**Table 1: Bacterial strains and their culture conditions used in this study**

| **Indicator organism** | **Culture Medium** | **Temp (°C)** + **Conditions** |
|---|---|---|
| *Fusobacterium nucleatum* DSM15643 | FAA | 37, Anaerobic |
| *Fusobacterium nucleatum* DSM19508 | FAA | 37, Anaerobic |
| *Fusobacterium nucleatum* DSM19509 | FAA | 37, Anaerobic |
| *Fusobacterium periodonticum* DSM19545 | FAA | 37, Anaerobic |
| *Escherichia coli* K12 | LB | 37, Aerobic |
| *Escherichia coli* ATCC25927 | LB | 37, Aerobic |
| *Clostridioides difficile* DPC6357 | FAA | 37, Anaerobic |
| *Streptococcus agalactiae* DPC7040 | BHI | 37, Anaerobic |
| *Streptococcus uberis* DPC4344 | BHI | 37, Anaerobic |
| *Streptococcus mutans* DPC6160 | BHI | 37, Anaerobic |
| *Streptococcus mutans* DPC6161 | BHI | 37, Anaerobic |
| *Streptococcus thermophilus* DPC5472 | BHI | 37, Anaerobic |
| *Streptococcus thermophilus* DPC5657 | BHI | 37, Anaerobic |
| *Streptococcus agalactiae* ATCC13813 | BHI | 37, Anaerobic |
| *Streptococcus simulans* APC3482 | BHI | 37, Anaerobic |
| *Listeria monocytogenes* DPC3564B | BHI | 37, Aerobic |
| *Listeria monocytogenes* DPC3853B | BHI | 37, Aerobic |
| *Listeria innocua* DPC3572 | BHI | 37, Aerobic |
| *Lactobacillus fermentum* DPC3320 | BHI | 37, Aerobic |
| *Lactobacillus plantarum* DPC6667 | BHI | 37, Aerobic |
| *Lactobacillus delbrueckii ssp. bulgaricus* DPC5383 | MRS | 37, Anaerobic |
| *Lactococcus lactis* NZ9700 | GM17 | 30, Aerobic |
| *Staphylococcus aureus* DPC7016 | BHI | 37, Aerobic |
| Methicillin-resistant *Staphylococcus aureus* DPC5654 | BHI | 37, Aerobic |
| *Staphylococcus epidermidis* DPC5990 | BHI | 37, Aerobic |

| | | |
|---|---|---|
| FAA, Fastidious Anaerobic Agar (Lab M, Lancashire, UK); BHI, Brain Heart Infusion; GM17, Glucose (0.5%) M17; (Difco Laboratories, Detroit, MI); LB, Luria-Bertani Medium; MRS, de Man, Rogosa, and Sharpe medium (Difco Laboratories, Detroit, MI); DSMZ, German Collection of Microorganisms and Cell Culture GmbH; ATCC, American Type Culture Collection; APC, Alimentary Pharmabiotic Centre. | | |

### Colony mass spectrometry of S. salivarius DPC6487

Fully grown colonies of *S*. *salivarius* DPC6487 were mixed with 50µl 2-propanol 0.1% TFA, vortexed three times and centrifuged at 14,000 rpm for 30 seconds. MALDI TOF mass spectrometry was performed on the cell free supernatant using an Axima TOF² MALDI-TOF mass spectrometer (Shimadzu Biotech, Manchester, UK). A 0.5µl aliquot of matrix solution (α-cyano 4-hydroxy cinnamic acid, 10 mg/ml in acetonitrile - 0.1% (v/v) TFA) was deposited onto the target and left for 5 seconds before being removed. The residual solution was allowed to dry and 0.5µl sample solution was deposited onto the pre-coated sample spot. 0.5µl of matrix solution was added to the deposited sample and allowed to dry. The sample was subsequently analysed in positive-ion linear mode.

### Purification of nisin G from S. salivarius DPC6487

Nisin G was purified from 2L of *S. salivarius* DPC6487 grown anaerobically in BHI for 24 hours corresponding to 10⁹ CFU/ml. The culture was centrifuged at 7,871 g for 20 minutes and cells separated from the supernatant. The cell pellet was resuspended in 300 ml of 70% 2 propanol 0.1% TFA (IPA) and stirred at room temperature for 3-4 hours. The resulting suspension was centrifuged again, and the supernatant retained for purification of the antimicrobial. An aliquot was assayed for antimicrobial activity against *L. bulgaricus* DPC5383 in an agar well diffusion assay (WDA) and MALDI TOF mass spectrometry (as previously outlined in these methods) of the cell extract confirmed the presence of the nisin G peptide mass (3405 Da). The IPA was removed by rotary evaporation and the sample applied to a 12 ml, 2 g Strata-E C18 SPE column pre-equilibrated (Phenomenex, Cheshire, UK) with methanol and water. The column was washed with 24 ml 30% ethanol and then 24 ml 70% 2-propanol 0.1 % TFA . A WDA using *L. bulgaricus* DPC5383 as an indicator showed that the antimicrobial activity was retained by the column and no activity was lost in the 30% ethanol wash, activity was eluted in the IPA wash. The C18 SPE eluent demonstrated increased antimicrobial activity suggesting that the nisin G concentration increased per mL. The IPA was removed from the C18 SPE IPA eluent by rotary evaporation (Buchi Labortechnik AG; Flawil, Switzerland) and the resulting sample applied to a semi prep Jupiter Proteo (10 × 250 mm, 4µM, 90Å) Reversed Phase HPLC column running a 30-50% acetonitrile 0.1% TFA gradient at 2.5 mL/min where buffer A was 0.1% TFA and buffer B was 100% acetonitrile 0.1% TFA. The eluent was monitored at 214 nm and fractions were collected at 1-minute intervals and again, antimicrobial activity was evaluated by WDA using *L. bulgaricus* DPC5383 as the indicator. Bioactive fractions were pooled and lyophilized using a Genevac lyophiliser (Suffolk, UK).

### Preparation and purification of nisin A

Nisin A was purified from nisinA^{®}P (Handary Sa; Brussels, Belgium) by Reversed Phase HPLC. Specifically, 60 mg of nisinA^{®}P powder was resuspended in Milli Q water to a concentration of 10 mg/ml. Aliquots of 2 ml were run on a semi preparative, Jupiter Proteo (10 x 250 mm, 4µ, 90Å), Reversed Phase HPLC column (Phenomenex, Cheshire, UK). An acetonitrile gradient was run at 25-45% where buffer A was 0.1% TFA and buffer B was 100% acetonitrile 0.1% TFA. Eluent was monitored at 214 nm and fractions collected at 30 second intervals. Nisin A-containing fractions were confirmed by MALDI TOF mass spectrometry by detection of the 3352 Da nisin A mass. Pure fractions were pooled and lyophilized using a Genevac lyophiliser (Suffolk, UK).

### Antimicrobial Activity Assays

Antimicrobial activity of *S. salivarius* DPC6487 and *L. lactis* NZ9700 against *F. nucleatum* DSM15643 and a range of indicator strains was determined by a deferred antagonism assay (Tagg *et al*., 1976). In brief, a fully cultured nisin-producer streak plate was overlayed with 0.75% agar seeded with the indicator microorganism and incubated according to the growth condition of the indicator microorganism. Inhibitory activity of purified nisin A and G was determined by well diffusion assay (WDA). 50 µl of 261mM nisin peptide (dissolved in molecular biology grade water) were added to wells of indicator plates and incubated according to the growth condition of the indicator microorganism (Table 9). Antimicrobial activity was determined by the presence of a zone of inhibition.

### Heat, pH and protease sensitivity assays

The stability of the antimicrobial secreted by *S. salivarius* DPC6487 to a variety of physio-chemical factors was examined. Initially, CFS was subjected to temperatures of 37, 60, 70, 80, 90 and 100°C for 10 minutes and the heat treated CFS evaluated for its antimicrobial activity against *L*. *bulgaricus* DPC5383 by WDA, as previously described. A pH stability test was performed by altering the pH of *S. salivarius* DPC6487 CFS to 2.0, 3.0, 5.0, 8.0, and 10.0 by addition of 1M HCL or 1M NaOH. Antimicrobial activity of the pH adjusted CFS against *L*. *bulgaricus* DPC5383 was evaluated by WDA. A proteinase sensitivity assay was performed by incubating the *S. salivarius* DPC6487 CFS with 20 mg/ml proteinase K (Sigma) at 37°C for 1 hour. CFS mixed with an equal volume of molecular biology grade water was included as a control. The antimicrobial activity of these CFSs were determined against *L. bulgaricus* DPC5383 by WDA.

### S. salivarius DPC6487 whole genome sequencing and analysis

Genomic DNA was extracted from *S. salivarius* DPC6487 culture cell pellets using a GenElute^{™} Bacterial Genomic DNA Kit (Sigma-Aldrich; Co. Wicklow, Ireland). The purity and concentration of genomic DNA was confirmed using the NanoDrop 1000 (ThermoFisher Scientific, Dublin, Ireland) and Qubit^{®} 2.0 Fluorometer (ThermoFisher Scientific, Dublin, Ireland) according to the respective protocols. DNA were prepared according to the Nextera XT DNA library preparation guide from Illumina and sequenced on an Illumina MiSeq (Teagasc, Moorepark Sequencing Facility). Quality trimming of the resulting raw FastQ files was performed using TrimGalore (v.0.6.0) (URL: https://www.bioinformatics.babraham.ac.uk/projects/trim_galore/), a wrapper script for cutadapt (v. 2.6) ) and FastQC (v. 0.11.8) with a q-score cut-off of 30 and minimum length after trimming of 105bp. Error correction and assembly in to contigs was performed using SPAdes (v. 3.14) in "isolate" run mode. The assembled contigs of the draft genome were annotated using Prokka (v. 1.14) with RNAmmer for rRNA prediction. BAGEL4 software, an automated bacteriocin mining tool was used to detect the presence of putative bacteriocin operons. Manual analysis of the contigs was then subsequently performed using the ARTEMIS genome browser. The putative bacteriocin gene clusters were manually annotated following sequence similarity analyses using the BLASTp algorithm and the non-redundant database provided by the NCBI (http://blast.ncbi.nlm.nih.gov). Multiple sequence alignment of nisin amino acid sequences was performed using ClustalW and the phylogenetic relationship was inferred using the Neighbourhood Joining method; evolutionary distances were computed using Poisson correction method and the tree was constructed with MEGAX with 1000 rounds of bootstrapping and visualised using ITOL.

### Results

### Characterisation of an unknown antimicrobial produced by S. salivarius DPC6487

The *S*. *salivarius* strains stored at -80°C in the Teagasc Culture Collection, Moorepark, Fermoy, Co Cork, Ireland, were evaluated for their potential anti*-Fusobacterium* activity. *S. salivarius* DPC6487 was isolated from a neonatal faecal sample as per the above. In this study, *S*. *salivarius* DPC6487 was found to demonstrate antimicrobial activity against the pathogen *F. nucleatum* DSM15643 **(****Figure 1A****).** The CFS of *S. salivarius* DPC6487 was evaluated for antimicrobial activity against the sensitive indicator *L. bulgaricus* DPC5383 in a WDA and distinct activity was evident **(****Figure 1B****)**. Subsequently, MALDI-TOF colony mass spectrometry of *S. salivarius* DPC6487 revealed the presence of a 3405 Da mass **(****Figure 1C****).**

The antimicrobial activity of CFS from *S. salivarius* DPC6487 remained stable at 37, 60, 70, 80, 90, 100, and 121°C **(****Figure 2A****).** Activity of the CFS also remained active at pH 2, 3, 5, 8, and 10, with greatest antimicrobial activity observed at pH 2 and 3 and decreasing from pH 5 to pH 10 **(Figure 211B).** Proteinase treatment of *S. salivarius* DPC6487 CFS resulted in the loss of antimicrobial activity and taken together the results indicate that the antimicrobial being produced was heat stable, proteinaceous in nature, and retained activity in acidic environments **(****Figure 2C****).**

### Whole genome sequencing of S. salivarius DPC6487 revealed a gene cluster encoding a natural nisin variant

Sequencing of *S*. *salivarius* DPC6487 yielded a 2,351,689 bp draft genome with an overall GC content of 39.75%. Blast analysis of the 16S rRNA sequence available on the draft genome confirmed a 99% identity to *S. salivarius* 16S rRNA gene sequences. Contig analysis via the bacteriocin mining tool BAGEL4 indicated the presence of a potential nisin variant. Sequencing analysis confirmed that the genome of *S. salivarius* DPC6487 harboured a gene predicted to encode a natural nisin variant that was designated in this study as nisin G. The putative novel nisin G peptide contains the following amino acid substitutions compared to nisin A: Ile4Thy, Ala15Val, Gly18Ala, Asn20His, Meth21Leu, His27Asn and His31Ile (Figure3). Nisin G is most similar to nisin Q (produced by L. lactis 61-14) with five amino acid substitutions: Ile4Thy, Gly18Ala, Asn20His, Val30Ile and His31Ile. Nisin G and nisin Q share three amino acid substitutions when compared to nisin A: Ala15Val, Meth21Leu, and His27Asn. Interestingly, nisin H, produced by *S. hyointestinalis,* shares no common amino acid substitutions with nisin G, however, amino acids at positions 18, 21 and 31 are changed in both variants. Nisin G demonstrates three unique amino acid variations when compared to all natural nisin variants, specifically Gly18Ala, Asp20His and His31Ile **(****Figure 3****).**

A phylogenetic comparison of all nisin variants demonstrated that respective streptococcal and lactococcal nisin variants group together. The *Blautia* derived nisin O₁₋₄ and salivaricin D are more distantly related. **(****Figure 4****).**

### Genomic analysis of S. salivarius DPC6487 identified genes putatively responsible for production of and immunity to nisin G

Genomic analysis identified nisin-related genes on two separate assembled contigs from the draft genome of *S. salivarius* DPC6487. Nisin genes nsgGBTCPRK were identified on a single contig of ~12.5kb in length. Putative nisin immunity determinants nsgFEG were found on the end of a separate large contig. Repeat regions and mobile element proteins surrounded both nisin-related regions and most likely contributed to the failure to assemble these respective contigs. The predicted nisin G pre-peptide sequence is composed of 57 amino acids containing a leader sequence of 23 amino acids. The nisin G mature peptide displays 78.95% and 84.21% amino acid similarity to nisin A and nisin H, respectively, and only 69.75% to salivaricin D. Therefore, nisin G was modelled against all other nisin variants and a comparison to nisin A is shown in **Figure 5****.**

### Purification of nisin G and nisin A

Nisin G was purified from a *S*. *salivarius* DPC6487 culture using Amberlite XAD16N solid-phase extraction, Sepharose cation exchange, C18 SPE, and reversed phase HPLC. Nisin A was purified from nisinA^{®}P powder (Handary Sa; Brussels, Belgium) using reversed phase HPLC.

Overall, it was observed that the purification of nisin G was less efficient compared to previous purifications of nisin A from *L. lactis* NZ9700 cell free supernatants. The yield for nisin G was 0.89mg/L of semi-pure peptide which is considerably lower to the usual yield of ~3 mg/L of nisin A pure peptide. It was noted from separate experiments that although cultures of nisin G and nisin A producers reach similar bacterial numbers (~10⁹ CFU/ml), CFS from the nisin A producer demonstrated 30-fold higher AU/ml compared to CFS from the nisin G producer when assessed against the indicator strain *L*. *bulgaricus* DPC5383 (20,480 AU/ml vs. 640 AU/ml, respectively) (data not shown). Taken together, it is possible that nisin G is produced in lower amounts than nisin A under the growth conditions used. Also, it is likely that the purification method that was used needs to be slightly optimized in order to increase the purity of nisin G peptide obtained. Antimicrobial activity of semi-pure nisin G and pure nisin A against *L. bulgaricus* DPC5383 at 261mM is shown in **Figure 6****.** Comparison of the size of the zones of inhibition that were observed indicate that the activity of nisin G is less than nisin A.

### Evaluating the anti-F. nucleatum activity of nisin G and nisin A

The antimicrobial activity of purified nisin G and nisin A at a concentration of 261mM were evaluated against *F. nucleatum* DSM15643 by means of WDA. Zones of inhibition were observed for both purified nisins. However, nisin A displayed increased antimicrobial activity against *F. nucleatum* DSM15643 with a zone of inhibition of 395.8mm² compared to nisin G, which generated a zone of 37.7mm². Interestingly, a larger zone of decreased cell concentration represented by a "halo effect" was observed surrounding the distinct zone of inhibition for nisin G **(****Figure 7A****).** When assayed by means of a deferred antagonism assay, nisin G-producing *S. salivarius* DPC6487 and nisin A-producing *L. lactis* NZ9700 demonstrated distinct antimicrobial activity against *F. nucleatum* DSM15643 **(****Figure 7B-C****).**

### Spectrum of inhibition of S. salivarius DPC6487 and L. lactis NZ9700

The spectrum of inhibition of nisin G-producing *S. salivarius* DPC6487 and nisin A-producing *L. lactis* NZ9700 was evaluated by deferred antagonism assay. *L. lactis* NZ9700 demonstrated antimicrobial activity against all Streptococcus species, however, *S. salivarius* DPC6487 was only active against related species S. thermophilus strains DPC5473 and DPC5657, and S. uberis DPC4344. Notably, *S. salivarius* DPC6487 showed increased activity against S. agalactiae ATCC13813 **(****Figure 8****).** In contrast to *L. lactis* NZ9700, *S*. *salivarius* DPC6487 showed no activity against *S*. *mutans* strains DPC6160 and DPC6161 or *S. simulans* APC3482. *S*. *salivarius* DPC6487 demonstrated no activity against *Lactobacillus* (except for *Lactobacillus delbrueckii* subsp. *bulgaricus*)*, Listeria,* or *Staphylococcus,* whereas, distinct antimicrobial activity was observed when *L. lactis* NZ9700 was used to target these genera. As nisin G-producing *S. salivarius* DPC6487 originally showed antimicrobial activity against *F*. *nucleatum* DSM15643, it was hypothesised that activity would be observed against other *Fusobacterium* species and *F. nucleatum* strains. *S. salivarius* DPC6487 and *L. lactis* NZ9700 both showed antimicrobial activity against *F. nucleatum* strains DSM15643, DSM19508, and DSM19509. *Fusobacterium periodonticum* DSM19545 was also susceptible to *S. salivarius* DPC6487 and *L*. *lactis* NZ9700, however, no activity was observed against *E*. *co*li K12 or *E. coli* ATCC25927. Interestingly, *L. lactis* NZ9700 demonstrated activity against *Clostridioides difficile* DPC6357, however, no activity was observed for *S. salivarius* DPC6487 against this strain (Table 2).

**Table 2: Indicators used in the inhibitory activity spectrum assessment of S. salivarius DPC6487 and L. lactis NZ9700 and the degree of inhibition. Zones of inhibition (mm) were measured around single colonies and relative sensitivity determined. +: zones of size <2mm, ++: zones of size 2-5mm, +++: zones of size >5mm.**

| | Inhibition | |
|---|---|---|
| | *S. salivarius* DPC6487 | *L. lactis* NZ9700 |
| Indicator | (nisin G producer) | (nisin A producer) |
| *Fusobacterium nucleatum* DSM15643 | ++ | ++ |
| *Fusobacterium nucleatum* DSM19508 | ++ | ++ |
| *Fusobacterium nucleatum* DSM19509 | ++ | ++ |
| *Fusobacterium periodonticum* DSM19545 | ++ | ++ |
| *Escherichia coli* K12 | - | - |
| *Escherichia coli* ATCC25927 | - | - |
| *Clostridoides difficile* DPC6357 | - | + |
| *Streptococcus mutans* DPC6160 | - | + |
| *Streptococcus mutans* DPC6161 | - | + |
| *Streptococcus thermophilus* DPC5472 | + | +++ |
| *Streptococcus thermophilus* DPC5657 | + | +++ |
| *Streptococcus agalactiae* ATCC13813 | +++ | +++ |
| *Streptococcus uberis* DPC4344 | + | + |
| *Streptococcus simulans* APC3482 | - | +++ |
| *Lactobacillus delbrueckii* subsp. *bulgaricus* DPC5383 | +++ | +++ |
| *Lactobacillus fermentum* DPC3320 | - | + |
| *Lactobacillus plantarum* DPC6667 | - | +++ |
| *Staphylococcus aureus* DPC7016 | - | ++ |
| *Methicillin-resistant Staphylococcus aureus* DPC5654 | - | +++ |
| *Staphylococcus epidermidis* DPC5990 | - | +++ |
| *Listeria monocytogenes* DPC3564B | - | +++ |
| *Listeria monocytogenes* DPC3853B | - | ++ |
| *Listeria innocua* DPC3572 | - | + |

| | | |
|---|---|---|
| DPC, Teagasc Culture collection; APC, APC Culture Collection; DMS, German Collection of Microorganisms; ATCC, American Type Culture Collection. | | |

### Discussion

CRC-associated *F. nucleatum* represents a potential therapeutic target and eradicating or suppressing the growth of this pathogen within the human gut microbiome may ultimately contribute to reducing or removing the overall risk of disease development.

In this study, *S*. *salivarius* DPC6487 was identified as having antimicrobial activity against *F*. *nucleatum* and, of particular interest, demonstrated increased potency compared to the salivaricin-producing *S. salivarius* DPC6993 against *L*. *bulgaricus.* Colony mass spectrometry of *S. salivarius* DPC6487 revealed a mass of 3405 Da, which indicated the potential secretion of a lantibiotic as similar lantibiotic masses have been reported. Additionally, heat, pH and proteinase sensitivity assays with *S*. *salivarius* DPC6487 CFS indicated that the antimicrobial being produced was likely proteinaceous.

Genome sequencing of *S*. *salivarius* DPC6487 revealed that the antimicrobial produced by *S. salivarius* DPC6487 was a novel nisin variant designated nisin G. The nisin variant nisin G contains 7 amino acid substitutions compared to nisin A and has three amino acid substitutions that are distinct from all other reported natural nisin variants; an alanine at position 18, a histidine at position 20, and an isoleucine at position 31. Notably, this is the first nisin reported from the species *S. salivarius.* Putative bacteriocin-like peptides were also found to be encoded in the genome of *S. salivarius* DPC6487.

As *S. salivarius* DPC6487 demonstrated antimicrobial activity against *F. nucleatum* and nisin G was subsequently discovered on its genome, it was postulated that nisin G was attributed to the anti-*F*. *nucleatum* activity observed. Nisin G was purified from *S. salivarius* DPC6487 and its antimicrobial activity against *F. nucleatum* DSM15643 was confirmed. Additionally, purified nisin A demonstrated increased antimicrobial activity against *F. nucleatum* DSM15643 compared to nisin G. This may be a consequence or the altered amino acids but also possibly due to the less efficient purification of nisin G. Indeed, site directed mutagenesis of the hinge region of nisin A resulting in either histidine at position 20 or leucine at position 21 (which are observed naturally in nisin G) reduced the bioactivity of the peptide against Gram-positive pathogens. Therefore, histidine in position 20 together with leucine in position 21 of the nisin G molecule may be contributing to the decreased potency observed compared to nisin A. Nonetheless, the nisin producers *S. salivarius* DPC6487 and *L*. *lactis* NZ9700 displayed distinct antimicrobial activity against *F. nucleatum* DSM15643. A limited number of studies have evaluated the antimicrobial activity of nisin against oral pathogens, which included *F. nucleatum, in vitro.* As *F. nucleatum* is now associated with diseases such as CRC gut microbiota-derived therapies against this emerging gut pathogen are now warranted.

Overall, *S. salivarius* DPC6487 showed a narrower spectrum of activity compared to *L. lactis* NZ9700, using the overlay method, with activity against just *Fusobacterium spp.* and other streptococci. Furthermore, *S. salivarius* DPC6487 showed no activity against *C. difficile* or *E. coli* strains tested in this study, indicating a potential narrow-spectrum against Gram-negatives. Narrow-spectrum antimicrobials are of particular interest as alternatives to antibiotics as they leave the surrounding microbiota unharmed. Both *S. salivarius* DPC6487 and *L. lactis* NZ9700 inhibited two additional *F. nucleatum* strains DSM19508 and DSM19509 and a *F. periodonticum* strain DSM19545, further supporting the *anti-Fusobacterium* potential of nisin and nisin-producers. Notably, *S. agalactiae* ATCC13813 was most susceptible to *S. salivarius* DPC6487 out of all streptococci included in the antimicrobial spectrum experiments. As invasive infections caused by *S. agalactiae* have been reported in pregnant women, new-borns and in adults with immunosuppressive diseases such as cancer and HIV, this finding suggests a possible application for *S. salivarius* DPC6487 to reduce the risk of *S. agalactiae* infection. As expected, the nisin A-producing *L*. *lactis* NZ9700 showed a broad spectrum of activity with activity against *Fusobacterium, Clostridioides, Streptococcus, Lactobacillus, Staphylococcus,* and *Listeria.* Furthermore, no activity was observed against Gram negative *E. coli* strains, as previously reported for nisin variants.

Nisin-producing bacteria represent potential candidates for the development of antimicrobial-producing probiotics, which may be utilized to target pathogenic bacteria, and consequently, lower the overall risk of disease development. The nisin G producing *S. salivarius* DPC6487 is a candidate for probiotic development with potential application to target CRC-associated *F. nucleatum,* therefore, improving CRC outcomes.

### EXAMPLE 2

### Streptococcus Salivarius DPC6487 for use as a probiotic strain

### Materials and Methods

### Isolation of S. salivarius DPC6487

### S. salivarius DPC6487 was isolated from a neonatal faecal sample

Samples included 32 neonates (2-12 days old). Samples were homogenized in maximum recovery diluent (MRD; Oxoid Ltd, Basingstroke, Hampshire, UK) as 10-fold dilutions, further diluted in MRD and appropriate dilutions spread-plated in duplicate on MRS agar and the plates were incubated anaerobically at 37°C. After 48 h, the colonies were enumerated and overlaid with 5 mL soft agar seeded with early stationary-phase cultures of two indicator strains, Listeria innocua DPC3572 (1×10⁷ CFUmL⁻¹ BHI agar, Oxoid Ltd) and Lactobacillus bulgaricus LMG 6901 (9×10⁶ CFUmL⁻¹ MRS agar), and incubated aerobically and anaerobically, respectively, at 37°C for 24 h. Colonies surrounded by zones of inhibition were then cultured in MRS broth and stocked at - 80°C for further characterization.

### Bacterial strains and growth conditions

All *S. salivarius* and *Streptococcus thermophilus* strains used for *in vitro* work in this study were cultivated under anaerobic conditions at 37 °C in Brain Heart Infusion (BHI, Difco Laboratories, Detroit, MI, USA). 1.5% w/v agar was added for preparation of agar. Anaerobic conditions were simulated using anaerobic jars and Anaerocult A gas packs (Merck, Darmstadt, Germany). All strains used in this study are shown in **Table 3.**

**Table 3: Bacterial strains used in this study.**

| **Strain** | **Isolation Source** |
|---|---|
| *Streptococcus salivarius* DPC6993 | Human faeces |
| *Streptococcus salivarius* DPC6383 | Human faeces |
| *Streptococcus salivarius* DPC6481 | Human faeces |
| *Streptococcus salivarius* DPC6385 | Human faeces |
| *Streptococcus salivarius* DPC6382 | Human faeces |
| *Streptococcus salivarius* DPC6487 | Neonatal faeces |
| *Streptococcus salivarius* DPC6988 | Human faeces |
| *Streptococcus salivarius* DPC6490 (ATCC 7073) | Human blood |
| *Streptococcus thermophilus* DPC5472 | Human faeces |
| *Streptococcus thermophilus* DPC5657 | Human faeces |

| | |
|---|---|
| DPC, Teagasc culture collection, Teagasc, Moorepark, Fermoy, Co. Cork, Ireland. | |

### Antibiotic susceptibility profiling

The Antibiotic susceptibility of *S. salivarius* DPC6487 and a range of other *S. salivarius* and *S. thermophilus* strains (Table 3) were assessed using VetMIC Lact-1 and Lact-2 plates (SVA, National Veterinary Institute, Upsala, Sweden). The VetMIC system comprises 96-well microtitre plates containing a range of clinical antibiotics with varying concentrations in powder form. The following antibiotics (with the range of concentration) were used in the susceptibility profiling of *S. salivarius* isolates; gentamycin (0.5-256µg/ml), kanamycin (2-1024µg/ml), streptomycin (0.5-256µg/ml), neomycin (0.5-256µg/ml), tetracycline (0.12-64µg/ml), erythromycin (0.016-8µg/ml), clindamycin (0.03-16µg/ml) and chloramphenicol (0.12-64µg/ml), ampicillin (0.03-16µg/ml), penicillin (0.03-16µg/ml), vancomycin (0.25-128µg/ml), quinupristin-dalfopristin (0.016-8µg/ml), linezolid (0.03-16µg/ml), trimethoprim (0.12-64µg/ml), ciprofloxacin (0.25-128µg/ml) and rifampicin (0.12-64µg/ml). Antibiotic susceptibility testing using the VetMIC assay was performed by using ISO sensitest medium combined with M17 medium at 90% and 10%, repetitively, (Oxoid, Hampshire, UK)) as outlined in ISO10932.

Colonies of each *S. salivarius* strain were picked from freshly streaked BHI agar plates and suspended in 5ml of sterile saline solution (0.85% NaCl solution) until an OD (625nm) between 0.16 and 0.2 was observed. Serial dilutions were carried out until diluted to 10⁻³ (1:1000) in the previously described ISO/M17 medium. 100µl of the diluent was delivered into each well of the Lact-1 and Lact-2 plates and incubated anaerobically at 37°C for 48h. The Minimum Inhibitory Concentration (MIC) was calculated based on the lowest antibiotic concentration where no visible growth was observed. Profiling of each isolate was performed in duplicate on vetMIC panels Lact-1 and Lact-2. MIC cut-off values for *S. thermophiles* published by the European Food Safety Authority (EFSA) were used as thresholds, as no published values for *S. salivarius* exists. *S. thermophiles* strains were used as controls.

### Bioinformatic analysis of S. salivarius DPC6487

The draft genome sequence of *S*. *salivarius* DPC6487 was analysed for prophages, mobile genetic elements and plasmids by BlastN via, PHASTER (https://phaster.ca/), prophage finder (https://omictools.com/prophage-finder-tool). Plasmids by BlastN via PlasmidFinder (https://cge.cbs.dtu.dk/services/PlasmidFinder/), mlplasmids, and the genome annotation. Clustered regularly interspaced short palindromic repeats (CRISPR) and cas genes were screened via CRISPRcasfinder (https://crisprcas.i2bc.paris-saclay.fr/). Antibiotic resistance genes were screened by blast analysis via the Comprehensive Antibiotic Resistance Gene Database (CARD) (https://card.mcmaster.ca/analyze/rgi), ResFinder (https://cge.cbs.dtu.dk/services/ResFinder/) and ARG-ONNOT. Potential virulence factors were screened by blast analysis via the Virulence Factor Database (VFDB) (http://www.mgc.ac.cn/VFs/main.htm). The pathogenic potential was assessed by PathogenFinder (https://cge.cbs.dtu.dk/services/PathogenFinder/). The genome sequences used in the comparative analysis were retrieved from the GenBank RefSeq database at the National Center for Biotechnology Information (NCBI) (http://www.ncbi.nlm.nih. gov/) (Table 2.). Proteins of *S. salivarius* DPC6487 and reference genomes were functionally categorised using the RAST annotation pipeline. Nucleotide and proteome comparisons were performed using OrthoANI and the SEED functional comparison tool, respectively.

**Table 4: Reference bacterial genomes used in the comparative genomic analysis with S. salivarius DPC6487.**

| **Strain** | **Accession** | **Source** | **Reference** |
|---|---|---|---|
| *S. salivarius* K12 | ALIF00000000 | Oral cavity | ³⁸ |
| *S. salivarius* M18 | AGBV00000000 | Oral cavity | ³⁹ |
| *S. salivarius* JIM8777 | FR873482 | Oral cavity | ⁴⁰ |
| *S. salivarius* HSISS4 | CP013216 | Faeces | ⁴¹ |

### DNA extractions and Conventional PCR

Genomic DNA of the *S. salivarius* strain was extracted from culture cell pellets using the GenEIute^{™} Bacterial Genomic DNA Kit (Sigma-Aldrich; Co. Wicklow, Ireland). The purity and concentration of genomic DNA was confirmed using the NanoDrop 1000 (ThermoFisher Scientific, Dublin, Ireland) and Qubit^{®} 2.0 Fluorometer (ThermoFisher Scientific, Dublin, Ireland) according to the respective protocols. Conventional PCR was used to amplify a 390bp long fragment of the *mefE* gene using the following primer sequences: *mefE* forward primer (SEQUENCE ID NO. 5), 5'-CCATCGACGTATTGGGTGCT-3'; *mefE* reverse primer (SEQUENCE ID NO. 6), 5'-AAACGGCTAAACTGGTCCCC-3'. Amplification was performed on the Applied Biosystems 2720 Thermocycler (ThermoFisher Scientific, Dublin, Ireland). Cycling conditions for the PCR analysis was as follows: 94°C for 1 min and 25 cycles of 94°C for 30 s, 55°C for 30 s and 72°C for 1 min, followed by 72°C for 5 min. Each PCR reaction contained 12.5µL BioMix Red (2X) (Bioline, Product# BIO-25006), forward and reverse primer (1µM), 10µl PCR grade water and 0.5 µL genomic DNA in a total reaction volume of 25µL. Negative controls comprised of PCR-grade water replacing genomic DNA.

### Results

### Comparative genomic analysis of S. salivarius

The genomes of *S. salivarius* DPC6487, *S. salivarius* DPC6993, *S. salivarius* HSISS4, *S. salivarius* JIM8777, *S. salivarius* K12 and *S. salivarius* M18 were included in genomic comparative analysis. The genome sizes ranged from 2.1 Mb to 2.4 Mb, with a GC content ranging from 39.5% to 40.9%.

**Table 5. Genomic features of the S. salivarius genomes included in the comparative analysis.**

| Strain | Isolation source | Genome Size (bp) | GC (%) | No. of CDSs |
|---|---|---|---|---|
| DPC6993 | Stool | 2,462,151 | 39.7 | 2319 |
| DPC6487 | Stool | 2,163,341 | 39.8 | 1989 |
| HSISS4 | Stool | 2,100,988 | 40.2 | 1903 |
| JIM8777 | Oral Cavity | 2,210,574 | 40.9 | 1979 |
| M18 | Oral Cavity | 2,142,944 | 39.6 | 1975 |
| K12 | Oral Cavity | 2,426,359 | 39.5 | 2257 |

| | | | | |
|---|---|---|---|---|
| DPC, Teagasc culture collection, Teagasc, Moorepark, Fermoy, Co. Cork, Ireland; bp, basepairs; GC, guanine cytosine; CDS, coding sequences. | | | | |

A phylogenetic analysis using the Randomized Axelerated Maximum Likelihood (RAxML) within the codon tree pipeline at Pathosystems Resource Integration Center (PATRIC) was performed based on the whole genome comparisons of *S. salivarius* strains DPC6993 and DPC6487 and reference *S. salivarius* genomes. Cross-genera protein families (PGFams) were used as homology groups. The analysis resulted in an alignment of 965 proteins, 292,603 amino acids and 877,809 nucleotides. Overall, the phylogenetic tree constructed showed that the DPC6487 strain most closely related to the gut isolate HSISS4 **(****Figure 9****).**

The proteome comparison of *S. salivarius* DPC6487 with HSISS4, JIM8777, K12, and M18 was visualised using the SEED proteome comparison tool. Protein regions of high similarities (largely >90%; indicated by green colour) between all comparative strains using DPC6487 **(****Figure 10.B****)** as reference strains are present, along with strain-strain similarities and strain-strain genomic gaps. Overall, 1,356 proteins were conserved in all *S. salivarius* strains.

The predicted proteins of *S. salivarius* strains were functionally annotated and categorised using the RAST sever. Subsystem analysis of genes assigned them into orthologous groups of proteins via the SEED. Overall, a similar number of proteins were assigned to each functional category across *S. salivarius* strains. The most common functional categories were related to housekeeping roles included protein metabolism, carbohydrate metabolism and transport and DNA metabolism, with a large subset of proteins assigned functional roles in virulence, disease, and defence **(Table 6).**

**Table 6: Distribution of subsystems of S. salivarius estimated by the RAST server**

| | ***S. salivarius* strains** | | | | | |
|---|---|---|---|---|---|---|
| **Subsystem category** | **DPC6993** | **DPC6487** | **HSISS4** | **JIM8777** | **K12** | **M18** |
| Cofactors, Vitamins, Prosthetic Groups, Pigments | 70 | 70 | 68 | 68 | 75 | 69 |
| Cell Wall and Capsule | 52 | 54 | 48 | 51 | 55 | 47 |
| Virulence, Disease and Defense | 32 | 35 | 32 | 31 | 36 | 31 |
| Potassium metabolism | 3 | 3 | 3 | 3 | 3 | 3 |
| Miscellaneous | 9 | 9 | 9 | 9 | 9 | 9 |
| Phages, Prophages, Transposable elements, Plasmids | 5 | 1 | 0 | 10 | 1 | 2 |
| Membrane Transport | 46 | 44 | 43 | 41 | 44 | 43 |
| Iron acquisition and metabolism | 8 | 7 | 7 | 7 | 7 | 11 |
| RNA Metabolism | 32 | 32 | 32 | 32 | 34 | 31 |
| Nucleosides and Nucleotides | 80 | 78 | 74 | 74 | 88 | 77 |
| Protein Metabolism | 131 | 107 | 119 | 121 | 127 | 125 |
| Cell Division and Cell Cycle | 4 | 4 | 4 | 4 | 4 | 4 |
| Regulation and Cell signaling | 14 | 12 | 11 | 14 | 13 | 11 |
| Secondary Metabolism | 8 | 4 | 4 | 4 | 6 | 6 |
| DNA Metabolism | 51 | 52 | 63 | 52 | 76 | 54 |
| Fatty Acids, Lipids, and Isoprenoids | 21 | 21 | 21 | 21 | 22 | 21 |
| Respiration | 13 | 13 | 13 | 13 | 13 | 15 |
| Stress Response | 18 | 19 | 18 | 21 | 18 | 18 |
| Amino Acids and Derivatives | 165 | 163 | 158 | 155 | 162 | 159 |
| Carbohydrates | 111 | 107 | 106 | 103 | 98 | 104 |

Genes present on mobile genetic elements such plasmids, prophages or phages are susceptible to horizontal gene transfer (HGT). Two potential prophage regions were identified in *S. salivarius* DPC6487 spanning genomic regions of 25.6Kb and 15.1Kb with 14 proteins encoded, however, these were classified as incomplete and demonstrated lower GC content (34.2% and 37.1%, respectively) than the genomic average of 39.8% **(Table 7).**

**Table 7. Summary of predicted prophage regions in S. salivarius DPC6487 genomes.**

| **Strain** | **Size** | **Completeness** | **No. of proteins** | **GC percentage** |
|---|---|---|---|---|
| DPC6487 | 25.6 Kb | Incomplete | 14 | 34.19% |
| | 15.1 Kb | Incomplete | 14 | 37.07% |

No complete plasmids were identified in the genomes of DPC6487 by *in silico* tools, however, plasmid associated genes were identified in DPC6487 and the plasmid sequence prediction tool mlplasmids (using a minimum posterior probability of 0.7 and minimum contig length of 1000 bp) predicted a number of contigs as plasmid associated. Blast analysis of contigs harbouring plasmid-associated genes revealed high nucleotide similarity (>95%) to previous characterised *S. salivarius* plasmid sequences, with most similarity to the mega-plasmid produced by *S. salivarius* ATCC 25975. Interestingly, the novel nisin G and its corresponding genetic organisation are located on two separate contigs in the initial assembly, both of which comprise genomic sequences with similarity to sequences of the ATCC 25975 plasmid.

### Evaluation of the potential of S. salivarius DPC6487 to contribute to antibiotic resistance by in vitro and in silico analysis

The antimicrobial susceptibility of *S. salivarius* DPC6487 to 17 clinical antibiotics was determined by the broth-dilution method via the VetMIC system and compared to the thresholds of *S*. *thermophilus* under EFSA guidelines, as no *S. salivarius* thresholds are published. *S*. *salivarius* DPC6487 was susceptible to all clinical antibiotics tested under EFSA guidelines. **(Table 8).**

**Table 8. Minimum Inhibitory Concentrations (MICs) of clinically used antibiotics for S. salivarius DPC6487.**

| | **MIC (µg/mL)** | |
|---|---|---|
| **Antibiotic** | ***EFSA Cut-off-*thermophilus*** | ***S. salivarius* DPC6487** |
| Gentamycin | 32 | 8 |
| Kanamycin | 64 | 64 |
| Streptomycin | 64 | 16 |
| Neomycin | Unpublished | 16 |
| Erythromycin | 2 | 1 |
| Clindamycin | 2 | 0.06 |
| Chloramphenicol | 4 | 2 |
| Ampicillin | 2 | 0.5 |
| Penicillin | Unpublished | 0.12 |
| Vancomycin | 4 | 0.5 |
| Quinupristin-dalfopristin | Unpublished | 1 |
| Linezolid | Unpublished | 1 |
| Trimethoprim | Unpublished | 8 |
| Ciprofloxacin | Unpublished | 2 |
| Rifampicin | Unpublished | 0.25 |
| Tetracycline | 4 | 1 |

| | | |
|---|---|---|
| ^{∗}*S. thermophilus* cut-off values are used as no *S. salivarius* values are published by EFSA. Duplicate experiments were performed with similar results. | | |

To better understand the potential of *S. salivarius* DPC6487 to contribute to antimicrobial resistance, the genome was screened for antibiotic resistance (ABR) genes using the resistance gene identifier (RGI) pipeline via the CARD and the ARG-ANNOT database. No antibiotic resistant determinants were identified in the genome of DPC6487 supporting the *in vitro* susceptibility of this strain to clinical antibiotics, as previously described.

### Confirmation of gene mefE in erythromycin resistant S. salivarius and its absence in erythromycin sensitive S. salivarius including strain DPC6487 by PCR

Erythromycin sensitive *S*. *salivarius* strains DPC6487 and DPC6383 produced no PRC product when targeting *mefE,* indicating its absence from both these genomes **(Figure 14).**

### Putative virulence factors

The potential risk of *S. salivarius* DPC6487 pathogenicity was assessed by screening the contigs of the draft genome for virulence factors by blast analysis against the VFDB and screening the genome annotations for homologues to characterised streptococcal virulence factors. No characterised Streptococcal toxins or superantigens were identified in DPC6487 genomes. Homologues to genes associated with virulence were identified by searching against the VFDB, however, these were mainly associated with adherence and enzymatic metabolism and essential roles in cellular housekeeping. Capsule proteins, choline-binding proteins, and Streptococcal glucosyltransferases were identified in DPC6487 genome. Also identified were homologues to one sortase A, one Streptococcal plasmin receptors, one Listeria adhesion protein, one Streptococcal enolase, one Pneumococcal surface antigen, and one trigger factor. BlastP analysis of the *lap* homologue resulted in hits to a streptococcal alcohol dehydrogenase and analysis of all other homologues demonstrated hits across the *Streptococcus* genus (including high similarity hits to GRAS strains *S. salivarius* K12 and M18 gene sequences). This indicates that *Streptococcus* core genes are likely being mischaracterised as virulence-associated. (Table 9).

**Table 9. Presence and absence of important virulence factors and virulence-associated genes in the genome of S. salivarius DPC6487.**

| **Virulence Factor** | **Virulence Category** | **Gene** | ***S. salivarius* strain** |
|---|---|---|---|
| | | | **DPC6487** |
| CAMP factor | Toxin | *cfa* | -ve |
| Streptolysin S | | *SagA* | -ve |
| Streptolysin O | | *slo* | -ve |
| Hyaluronate lyase | | *hyl* | -ve |
| Nicotin adenine dinuclutide glycohydrolase | | *nga* | -ve |
| C3 family ADP-ribosyltransferase | | *SpyA* | -ve |
| Streptococcal pyrogenic exotoxin A | Superanigen | *SpeA* | -ve |
| Streptococcal pyrogenic exotoxin B | | *SpeB* | -ve |
| Streptococcal pyrogenic exotoxin C | | *SpeC* | -ve |
| Streptococcal pyrogenic exotoxin G | | *SpeG* | -ve |
| Streptococcal pyrogenic exotoxin H | | *SpeH* | -ve |
| Streptococcal pyrogenic exotoxin I | | *SpeI* | -ve |
| Streptococcal pyrogenic exotoxin J | | *SpeJ* | -ve |
| Streptococcal pyrogenic exotoxin K | | *SpeK* | -ve |
| Streptococcal pyrogenic exotoxin L | | *SpeL* | -ve |
| Streptococcal pyrogenic exotoxin M | | *SpeM* | -ve |
| Streptococcal superantigen A | | *SSA* | -ve |
| Streptococcal metogenic exotoxin Z | | *SmeZ* | -ve |
| M-protein | Adherence | *emm* | -ve |
| Protein H | | *sph* | -ve |
| Fibrinogen binding protein | | *fba* | -ve |
| Fibrotectin-binding protein (protein F) | | *prtF* | -ve |
| Protein G-related alpha 2 macroglobulin binding protein | | *grab* | -ve |
| Fibronectin-binding protein | | *FBP* | -ve |
| Serum opacity factor | | *SOF* | -ve |
| Collagen-like surface protein | | *SclB* | -ve |
| Choline-binding protein | | fbp54 | +ve |
| Choline-binding protein | | *cbpD* | +ve (2) |
| Sortase A | | srtA | +ve |
| Streptococcal glucosyltransferase | | *gtfD* | +ve |
| Streptococcal glucosyltransferase | | gtfG | +ve(2) |
| Streptococcal plasmin receptor/GAPDH | | plr/gapA | +ve |
| Listeria adhesion protein (Listeria) | | lap | +ve |
| Serine endopeptidase | Enzyme | *ScpC* | -ve |
| Hyaluronan synthase | | *HasA* | -ve |
| Streptodornase B | | *SdaB* | -ve |
| Streptococcal enolase | | eno | +ve |
| Streptokinase | Protease | *Ska* | -ve |
| Immunoglobulin G-endopeptidase | | *IdeS* | -ve |
| C5a peptidase | | *ScpA* | -ve |
| Trigger factor | | tig/ropA | +ve |
| Capsule | Antiphagocytosis | cps1 | +ve |
| Capsules | Immune invasion | - | +ve (20) |
| Streptococcal inhibitor of complement | | *SIC* | -ve |
| Pneumococcal surface antigen A | Magnesium uptake | psaA | +ve |

| | | | |
|---|---|---|---|
| ^{∗}Numbers in brackets indicate number of gene copies identified | | | |

### Discussion

Probiotic bacteria hold potential application with a view to biomedical and functional food development. *S. salivarius* DPC6487 represents a candidate for probiotic development with potential application to suppress the growth of *F. nucleaum* in the human colon, which may reduce the overall risk of CRC development. In this study, a genomic safety assessment was performed based on draft genome sequences of *S. salivarius* DPC6487. Furthermore, the antibiotic susceptibility profile of the strain was determined *in vitro.*

A comparative genomic analysis indicated that *S*. *salivarius* DPC6487 shared similar genomic features with *S. salivarius* reference genomes HSISS4, JIM8777, K12, and M18. DPC6487 displayed a similar GC content, length (bp) and number of CDSs to reference genomes. ANI comparisons resulted in >95% ANI between all *S. salivarius* genomes. A proteome comparison demonstrated a high degree of protein conservation. Overall, 1,356 proteins were conserved in all strains included in the comparative analysis, which indicates considerable variability in *S. salivarius* gene content as previously reported.

The stability of genomes is a key characteristic for probiotic bacteria. Absence of mobile genetic elements, prophages or phages from probiotic bacteria is desirable, particularly from a food industry perspective, and their presence may contribute to genomic instability. Although prophage like elements were identified in the genome of DPC6487, no complete prophage was detected.

Key defence mechanisms against mobile genetic elements are restriction modification systems and CRISPR-Cas systems. Although, CRISPR-associated sequences were identified in the genome of DPC6487, no CRISPR cas genes were discovered. Complete type-I R-M systems were discovered in the DPC6487 genome. The presence of these R-M systems may provide a significant barrier to HGT events, and therefore, may play a significant role in the stability of DPC6487 genome. Interestingly, a type-I toxin/antitoxin (TA) system was identified within the type-I RM genomic region discovered in DPC6487. The role of TA systems is proposed to include genomic stabilization, phage infection defence and stress modulation.

The EFSA guidance on assessment of bacterial susceptibility to antimicrobials for human and veterinary use was published in 2012, however, no MIC thresholds for *S. salivarius* were reported, therefore, the inventors used threshold values available for the closely related species *S. thermophilus.* DPC6487 was susceptible to all clinically relevant antibiotics tested, which suggested an inability to contribute to antibiotic resistance.

Candidate probiotic strains must be free of virulence factors which may contribute to their pathogenicity. No characterised Streptococcal toxins or superantigens were identified in the genome DPC6487. Capsule proteins characterised as contributing to immune invasion were identified, however, expression of capsules by streptococci has been shown to be a significant occurrence of our commensal microbiota. The remaining virulence factors were mainly associated with adherence and surface factors, which are not likely to contribute to the virulence of *S. salivarius.* It should be noted that, both pathogens and commensals use similar strategies to survive passage through the harsh conditions of the GI tract and colonise the gut and many of these strategies are often characterised in pathogenic microbes, therefore are often mischaracterised as virulence factors, and thus, represent "niche-factors".

In conclusion, this study compared the genome of *S. salivarius* DPC6487 to reference *S. salivarius* genomes, including the previously characterised as safe strains K12 and M18. *S. salivarius* DPC6487 was susceptible all clinical antibiotics under EFSA threshold values for *S. thermophilus* and no antibiotic resistance determinants were discovered on its genome. No characterised streptococcal virulence factors were found in DPC6487.

Equivalents

## Claims

1. An isolated *Streptococcus salivarius* strain, **characterised in that** the strain is capable of inhibiting growth of *Fusobacterium spp.* and which produces an antimicrobial peptide of SEQUENCE ID NO 1 or a variant thereof, in which the variant has 1 to 3 amino acid changes compared with SEQUENCE ID NO. 1.

2. The isolated strain of Claim 1, **characterised in that** the strain is isolated from the human, preferably neonatal human, gastrointestinal (GI) tract.

3. The isolated strain of Claim 1 or 2, wherein the *Fusobacterium spp.* is selected from *Fusobacterium nucleatum and Fusobacterium periodontium.*

4. A *Streptococcus salivarius* DPC6487 strain as deposited with the National Collection of Industrial Food and Marine Bacteria under the Accession No. NCIMB 43881.

5. An antimicrobial peptide expressed by the strain of any one of the preceding claims.

6. An antimicrobial peptide comprising the sequence of SEQUENCE ID NO. 1, or a variant thereof, in which the variant has 1 to 3 amino acid changes compared with SEQUENCE ID NO. 1.

7. The antimicrobial peptide of Claim 6, which is capable of inhibiting growth of *Fusobacterium spp.*

8. A composition comprising the strain of any one of Claims 1 to 3, the strain of Claim 4 or the antimicrobial peptide of any one of Claims 5 to 7.

9. The composition of Claim 8, wherein the composition is formulated as an antibacterial or antibiotic formulation and/or formulated for topical administration.

10. A probiotic comprising the strain of any one of Claims 1 to 3, the strain of Claim 4 or the antimicrobial peptide of any one of Claims 5 to 7.

11. The strain of any one of Claims 1 to 3, the strain of Claim 4, the antimicrobial peptide of any one of Claims 5 to 7, or the composition of any one of Claims 8 to 9, for use as a medicament.

12. The strain of any one of Claims 1 to 3, the strain of Claim 4, the antimicrobial peptide of any one of Claims 5 to 7, or the composition of any one of Claims 8 to 10, for use in treating or preventing a disease or condition **characterised by** growth of *Fusobacterium* spp.

13. The strain, the antimicrobial peptide or the composition for use of Claim 12, wherein the disease or condition is one associated with the gastrointestinal (GI) tract, preferably the distal colon.

14. The strain, the antimicrobial peptide or the composition for use of Claim 13, wherein the disease or condition is one or more selected from the group comprising colorectal cancer (CRC), inflammatory bowel disease (IBD) and appendicitis.

15. The strain of any one of Claims 1 to 3, the strain of Claim 4, the antimicrobial peptide of any one of Claims 5 to 7, or the composition of any one of Claims 8 to 10, for use in treating or preventing a vaginal infection **characterised by** growth of *S. agalactiae* in a subject.
